# EUROPÄISCHE PATENTSCHRIFT

(11) **EP 3 682 242 B1**
(45) Veröffentlichungstag und Bekanntmachung des Hinweises auf die Patenterteilung: **05.03.2025**
(21) Anmeldenummer: 18769647.1
(22) Anmeldetag: 11.09.2018
(51) Int. Cl.: G01N 33/569

(54) **VERFAHREN ZUM NACHWEIS AKUTER BORNA DISEASE VIRUS (BDV) INFEKTIONEN UND EINES DIAGNOSTISCHEN KITS HIERFÜR, INSBESONDERE IN KOMBINATION MIT VERFAHREN ZUR UNTERSCHEIDUNG AKUTER VON CHRONISCHEN UND RUHENDEN BDV-INFEKTIONEN UND DIAGNOSTISCHE KITS HIERFÜR**
METHOD FOR DETECTING ACUTE BORNA DISEASE VIRUS (BDV) INFECTIONS, AND DIAGNOSTIC KIT THEREFOR, IN PARTICULAR IN COMBINATION WITH METHODS FOR DISTINGUISHING ACUTE FROM CHRONIC AND LATENT BDV INFECTIONS, AND DIAGNOSTIC KITS THEREFOR
PROCÉDÉ DE DÉTECTION DU VIRUS DE LA MALADIE BORNA AIGÜE (BDV) ET TROUSSE DE DIAGNOSTIC ASSOCIÉE, NOTAMMENT EN ASSOCIATION AVEC DES PROCÉDÉS POUR DISTINGUER LES INFECTIONS BVD AIGÜES DES INFECTIONS BVD CHRONIQUES ET DORMANTES ET TROUSSES DE DIAGNOSTIC ASSOCIÉES

(30) Priorität: 12.09.2017 DE 102017121046
(43) Veröffentlichungstag der Anmeldung: 22.07.2020
(73) Patentinhaber: Ludwig, Hanns, 14163 Berlin (DE); Bode, Liv, 14163 Berlin (DE)
(72) Erfinder: Ludwig, Hanns, 14163 Berlin (DE); Bode, Liv, 14163 Berlin (DE)
(74) Vertreter: Kröncke, Rolf
(86) Internationale Anmeldenummer: PCT/EP2018/074415
(87) Internationale Veröffentlichungsnummer: WO 2019/052990

(56) Entgegenhaltungen:
- L BODE ET AL: "Borna disease virus-specific circulating immune complexes, antigenemia and free antibodies-the key marker triplet determining infection and prevailing in severe mood disorders", MOLECULAR PSYCHIATRY, vol. 6, no. 4, 1 July 2001 (2001-07-01), GB, pages 481 - 491, XP055514102, ISSN: 1359-4184, DOI: 10.1038/sj.mp.4000909
- LIV BODE: "Human Bornavirus infection - towards a valid diagnostic system", APMIS, vol. 116, no. 116, 1 June 2008 (2008-06-01), DK, pages 21 - 39, XP055514105, ISSN: 0903-4641, DOI: 10.1111/j.1600-0463.2008.000m3.x
- L. BODE ET AL: "Borna Disease Virus Infection, a Human Mental-Health Risk", CLINICAL MICROBIOLOGY REVIEWS., vol. 16, no. 3, 1 July 2003 (2003-07-01), US, pages 534 - 545, XP055514090, ISSN: 0893-8512, DOI: 10.1128/CMR.16.3.534-545.2003
- HSU T A ET AL: "Borna disease virus p24 and p38/40 synthesized in a baculovirus expression system: virus protein interactions in insect and mammalian cells", VIROLOGY, ELSEVIER, AMSTERDAM, NL, vol. 204, no. 2, 1 November 1994 (1994-11-01), pages 854 - 859, XP009508573, ISSN: 0042-6822, DOI: 10.1006/VIRO.1994.1608
- L BODE ET AL: "Borna disease virus-specific circulating immune complexes, antigenemia and free antibodies-the key marker triplet determining infection and prevailing in severe mood disorders", MOLECULAR PSYCHIATRY, vol. 6, no. 4, 1 July 2001 (2001-07-01), GB, pages 481 - 491, XP055514102, ISSN: 1359-4184, DOI: 10.1038/sj.mp.4000909
- LIV BODE: "Human Bornavirus infection - towards a valid diagnostic system", APMIS, vol. 116, no. 116, 1 June 2008 (2008-06-01), DK, pages 21 - 39, XP055514105, ISSN: 0903-4641, DOI: 10.1111/j.1600-0463.2008.000m3.x
- L. BODE ET AL: "Borna Disease Virus Infection, a Human Mental-Health Risk", CLINICAL MICROBIOLOGY REVIEWS., vol. 16, no. 3, 1 July 2003 (2003-07-01), US, pages 534 - 545, XP055514090, ISSN: 0893-8512, DOI: 10.1128/CMR.16.3.534-545.2003
- HSU T A ET AL: "Borna disease virus p24 and p38/40 synthesized in a baculovirus expression system: virus protein interactions in insect and mammalian cells", VIROLOGY, ELSEVIER, AMSTERDAM, NL, vol. 204, no. 2, 1 November 1994 (1994-11-01), pages 854 - 859, XP009508573, ISSN: 0042-6822, DOI: 10.1006/VIRO.1994.1608

## Beschreibung

In einem ersten Aspekt richtet sich die vorliegende Erfindung auf ein Verfahren zum Nachweis akuter Borna Disease Virus (BDV) Infektionen. Erfindungsgemäß wird dabei das Vorliegen von Heterodimeren aus p24 BDV-Phosphoprotein und p40 BDV-Nukleoprotein in einer Probe mittels Sandwich-ELISA wie in den Ansprüchen ausgeführt bestimmt. Dieses Bestimmen umfasst dabei, dass spezifische, monoklonale Primärantikörper sowohl gegen das p24 BDV-Phosphoprotein als auch gegen das p40 BDV-Nukleoprotein eingesetzt werden.

In einem zweiten Aspekt wird die Verwendung eines diagnostischen Kits für einen Sandwich-ELISA zum Nachweis akuter BDV-Infektionen bereitgestellt. Dieses diagnostische Kit weist wie in den Ansprüchen ausgeführt einen ersten primären Antikörper und einen zweiten primären Antikörper auf, wobei einer dieser primären Antikörper gegen das p24 BDV-Phosphoprotein gerichtet ist, während der andere primäre Antikörper gegen das p40 BDV-Nukleoprotein gerichtet ist, mit mindestens einem sekundären Antikörper, der mit einem Reportermolekül markiert ist, Mittel zum Immobilisieren eines primären Antikörpers an eine Oberfläche sowie Anleitungen zur Durchführung des erfindungsgemäßen Verfahrens.

In einem dritten Aspekt richtet sich die vorliegende Erfindung darauf, akute BDV-Infektionen von chronischen und ruhenden Infektionen zu unterscheiden, indem das erfindungsgemäße Verfahren und die damit bereitgestellte Verwendung des diagnostischen Kits einerseits kombiniert wird mit einem Verfahren zum Nachweis von BDV Infektionen, bei denen die frei zirkulierenden Immunkomplexe (CIC) aus BDV Antigenen und hieran angelagerten spezifischen Antikörpern durch immunologische Tests nachgewiesen werden wie zum Beispiel in der DE 19758017 C2 beschrieben, und indem andererseits weiterhin ein Verfahren zum Nachweis von BDV-Infektionen, bei denen frei zirkulierende BDV-Antikörper im infizierten Wirt durch immunologische Tests nachgewiesen werden, genutzt wird. Entsprechend umfassen die Verwendung einfindungsgemäßer diagnostischer Kits Mittel zum Nachweis von BDV-CIC und ein entsprechendes diagnostisches Kit zum Nachweis von BDV-Antikörpern.

Schließlich wird die Verwendung des erfindungsgemäßen Verfahrens für die Diagnose akuter BDV-Infektionen und/oder deren Verlaufskontrolle bereitgestellt. Insbesondere in Kombination mit den Verfahren zum Nachweis von BDV-CIC und BDV-Antikörpern wird das erfindungsgemäße Verfahren zur Differenzierung akuter BDV-Infektionen von chronischen und ruhenden Infektionen und/oder deren Verlaufskontrolle und dem Ausschluss einer BDV-Infektion bereitgestellt. Es ist für Menschen und Tiere gleichermaßen geeignet. In welchem Zustand sich die Infektion befindet und ihr Verlauf hat unmittelbare Auswirkungen auf die Möglichkeit einer antiviralen Therapie bei klinisch erkrankten Individuen.

### Stand der Technik

Das Borna- Virus (englisch Borna Disease Virus; BDV bzw. BoDV-1 nach neuester taxonomischer Bezeichnung) ist taxonomisch das Leitvirus der Spezies *"Mammalian 1 bomavirus"* in der Virusfamilie *"Bornaviridae",* die zur Ordnung der *"Mononegavira*les" gehört. Seine genetisch zu mehr als 95% identischen Virusstämme infizieren Gehirn und Blutzellen beim Menschen und vielen Säugetieren und sind weltweit verbreitet. Beim Pferd ist es der Erreger der Bornaschen Krankheit. Borna- Viren sind behüllte Viren (90 nm Durchmesser) mit einer negativ-strängigen nicht-segmentierten einzelsträngigen (NSS) RNA von 8,910 Kilobasen, die für sechs Gene kodiert. Borna-Viren vermehren sich im Kern der Wirtszelle und gehören zu den entwicklungsgeschichtlich ältesten Viren, die seit mindestens 40 Millionen Jahren mit den Primatenvorfahren koexistieren und sogar in deren Erbgut bis zum Menschen dauerhafte Spuren hinterlassen haben. Heute kommen BDV Infektionen bei vielen Nutz- und Haustieren (u.a. Pferd, Schaf, Rind, Katze) und dem Menschen vor. Sie verbleiben lebenslang im infizierten Organismus (persistente Infektion ohne Zellzerstörung), befallen bevorzugt den entwicklungsgeschichtlich alten Teil des Gehirns (limbisches System) und sind an Verhaltens- und Stimmungsänderungen beteiligt.

Es wird davon ausgegangen, dass jeder dritte Mensch im Erwachsenenalter in Deutschland unbemerkt, d.h. ohne klinische Symptome, mit Borna- Viren infiziert ist (Bode and Ludwig, 2003). Bei jedem sechsten Infizierten (16-17%) besteht ein erhöhtes Risiko, im Laufe des Lebens an einer mentalen Störung zu erkranken. Bezogen auf die gesamte Bevölkerung hat jeder Zwanzigste (5 von 100 Menschen; 5%) ein erhöhtes Krankheitsrisiko. Als bedeutendster Risikofaktor wird chronischer Stress vermutet, der langfristig das Immunsystem schwächt und die Aktivierung ruhender Borna-Viren begünstigt.

Das Borna- Virus befällt bevorzugt die Nervenzellen des Limbischen Systems des Gehirns. Diese zentralen Hirnbereiche regeln Verhalten und Gefühle und sind an Lernprozessen und Gedächtnisbildung beteiligt. Eine Vermehrung des Virus erfolgt primär in Nervenzellen und kann sich über deren Fortsätze im gesamten Nervensystem ausbreiten. Dabei werden die Virusproteine N (p40 Nukleoprotein) und P (p24 Phosphoprotein) (Antigene) im Überschuss gebildet. Im Gehirn symptomatischer Patienten (z.B. bei Depression) tragen sie zu Funktionsstörungen des Gleichgewichts der Gehirnbotenstoffe bei (tierexperimenteller Nachweis).

BDV Infektionen sind beim Tier mit phasenhaften Verhaltensstörungen einschließlich Apathie, Aufmerksamkeitsverlusten und Bewegungskoordinationsstörungen assoziiert. Beim Menschen wurden akute oder chronische Borna-Virus Infektionen viel häufiger z.B. bei akuten depressiven Episoden, bei Zwangserkrankungen und beim chronischen Müdigkeitssyndrom gefunden als bei gesunden Menschen. Deswegen wird seit Jahren eine Mitbeteiligung der Borna-Viren an dem sehr komplexen Krankheitsgeschehen vermutet, die aber noch als umstritten gilt (Bode and Ludwig, 2003). Für die Beteiligung spricht der nachweisliche Nutzen einer antiviralen Therapie mit Amantadin. Endogene rezidivierende Depressionen, unipolar oder bipolar, gehören mit mindestens 5% Lebenszeitprävalenz zu den bedeutenden psychiatrischen Erkrankungen in Deutschland und weltweit.

Es besteht daher ein Bedarf an zuverlässigen Diagnosesystemen zur Erfassung von BDV-Infektionen, sowohl bei der Kontrolle der Infektionsphasen bei Erkrankten und zur Verlaufs- und Therapiekontrolle, als auch zur Erfassung unerkannt infizierter asymptomatischer Träger, bevölkerungsbezogen aus epidemiologischen Gründen oder personenbezogen zur Ermittlung des individuellen Erkrankungsrisikos.

Für die Beurteilung der Infektionsphasen (akut, chronisch, ruhend) hat sich die erhöhte Expression von BDV-Antigenen (N und P) im Blut als der entscheidende Aktivitätsparameter erwiesen. Er korreliert gut mit klinischer Erkrankung (Mensch und Tier), ist quantitativ messbar und wichtig zur Einschätzung des klinischen Verlaufs. Die Antigen-Bestimmung kann auch nicht durch den Nachweis der Virusnukleinsäure über Amplifikation mit nested-RT-PCR oder Realtime RT-PCR in den PBMCs ersetzt werden, da hierdurch nur das Virus selbst aber nicht dessen Aktivität bestimmt werden kann. Heutzutage erfolgt die Diagnose von Borna -Virus Infektionen vielerorts dennoch mit Nachweis des viralen Genoms oder ist serologisch auf den Nachweis von Antikörpern mittels diverser serologischer Methoden beschränkt. Allerdings ist ein negativer Nachweis von spezifischen Antikörpern allein kein geeignetes Instrument, um eine BDV Infektion auszuschließen, da freie Antikörper während einer Antigen-freisetzenden Vermehrungsphase in Form von zirkulierenden Immunkomplexen (CIC) im Serum des Infizierten gebunden werden und zeitweise unter die Nachweisgrenze sinken können.

In den letzten Jahren wurden diagnostische Verfahren auf Basis von ELISA Techniken durchgeführt, bei denen BDV-spezifische Immunkomplexe (CICs) sowie virale Proteine und gegebenenfalls frei zirkulierende Antikörper gemessen werden (Bode et al., 2001). Hierbei werden spezifische monoklonale Antikörper eingesetzt. Die Aminosäuresequenzen des BDV Nukleoproteins p40 und des Phosphoproteins p24 sind bekannt, seit 1994 die ersten beiden vollständigen Genome von zwei BDV-Virusstämmen sequenziert wurden (Briese et al., 1994; Cubitt et al., 1994). Für das Nukleoprotein konnte ein Ribbonmodell der dreidimensionalen Struktur erstellt werden (Rudolph et al., 2003).

Die Schlüsselreagenzien für die BDV-ELISA-Teste, die monoklonalen Antikörper W1 (anti-N) und Kfu2 (anti-P) waren in ihren Basiseigenschaften charakterisiert (Ludwig et al., 1993). Die BDV- CICs sind die am häufigsten nachweisbaren Infektionsmarker und stellten sich bisher als optimal für entsprechende Diagnostiktests heraus.

Darauf basierend betrifft die DE 19758017 C2 entsprechende Verfahren zum Nachweis von BDV Infektionen, bei denen die frei zirkulierenden Immunkomplexe aus BDV Antigenen und hieran angelagerte spezifische Antikörper durch immunologische Tests nachgewiesen werden. Dieses Schutzrecht betrifft weiterhin ein entsprechendes diagnostisches Kit, wie eingangs erwähnt. Eine Erweiterung hiervon findet sich in der DE 19860255 C2 gerichtet auf ein Verfahren zum Nachweis von BDV Infektionen, wobei der Antigennachweis in einer Plasmaprobe, Liquor oder Urin durchgeführt wird.

Entsprechende Bluttests auf BDV- CIC finden Anwendung. Die Nutzung des Immunkomplexes (CIC), der nur nachweisbar ist, wenn die Viren sich in Phasen vermehrt haben, ist gut geeignet, im positiven Fall chronische Infektionen anzuzeigen. Der zusätzliche frühere Antigentest wies im positiven Fall auf einen Schub hin. Ein negativer CIC-Test benötigte den zusätzlichen Antikörper-ELISA, um eine ruhende Infektion nachzuweisen oder die Infektion auszuschließen.

Die derzeitigen Diagnosesysteme und Verfahren beruhen auf dem Nachweis von CIC, wobei auf Basis eines Sandwich-ELISA mit monoklonalen Antikörpern aus einer ersten Spezies der Antigenanteil im CIC gebunden wird und dann der Antikörperteil des gebundenen CIC mit einem enzymmarkierten sekundären Anti-Antikörper aus einer anderen Spezies und einer der relativen Menge des CIC in der Probe entsprechenden Substratreaktion (Farbumschlag) nachgewiesen wird.

Der CIC-ELISA hat sich als Basistest zum Nachweis einer chronischen BDV-Infektion bewährt. Für den Nachweis der akuten BDV-Infektion, der für klinisch Erkrankte besonders wichtig ist, ist dieser Test alleine nicht geeignet. Der bisherige Antigen-Test ist eingeschränkt durch hohes Probenvolumen und limitierte Ressourcen und damit auf längere Sicht nicht als diagnostisches Kit einsetzbar.

Hsu T.A., et. al., 1994, Virology, 204, 854 - 859 beschreibt Borna Disease Virus p24 und p38/40 Synthese in ein Baculovirus Expressionssystem.

Die vorliegende Erfindung schließt diese Lücke durch Bereitstellung eines komplett neuen Testverfahrens für akute BDV-Infektionen. Durch Kombination mit CIC-Test und Antikörpertest ist das neue Verfahren in der Lage, akute, chronische und ruhende Infektionen zu unterscheiden und die BDV-Diagnostik damit mit hohem Qualitätsniveau auf eine bisher unerreichte Aussagekraft zu heben.

Die vorliegende Erfindung stellt ein komplett neues Testverfahren für akute BDV-Infektionen bereit. Die spezifischen Bindungsprofile der in den Verfahren gemäß DE 19758017 C2 eingesetzten monoklonalen Antikörpern "W1" und "Kfu2", die jeweils potente Konformationsepitope auf dem BDV-N- bzw. P-Protein erkennen, konnten inzwischen identifiziert werden (Bode, 2008). Die vorliegende Erfindung zielt auf die Erkennung der aktiven, phosphorylierten Form des P-Proteins und von Heterodimeren N/P. Die hier beschriebenen monoklonalen Antikörper sind daher für das neue Testverfahren und die damit kombinierten Testverfahren besonders gut geeignet.

### Beschreibung der Erfindung

Die Aufgabe der vorliegenden Erfindung ist die Bereitstellung eines Verfahrens zum Nachweis akuter BDV Infektionen sowie entsprechender Verwendung diagnostischer Kits auf Basis von ELISA, insbesondere Sandwich ELISA Techniken. Eine weitere Aufgabe ist die Bereitstellung verbesserter Verfahren zur Differenzialdiagnostik.

In einem ersten Aspekt richtet sich die Erfindung dabei auf ein Verfahren zum Nachweis akuter Borna Disease Virus (BDV) Infektionen gemäß Anspruch 1, umfassend den Schritt des Bestimmens des Vorliegens von Heterodimeren aus p24 BDV-Phosphoprotein (Seq. ID Nr. 2) und p40 BDV-Nukleoprotein (Seq. ID Nr. 1) in einer Probe mittels eines Sandwich-ELISA, wobei ein erster primärer Antikörper immobilisiert vorliegt und ein zweiter primärer Antikörper nicht immobilisiert nach Inkubation der Probe mit dem immobilisierten ersten Antikörper hinzugefügt wird, wobei der erste primäre Antikörper gegen das p24 BDV-Phosphoprotein gerichtet ist und der zweite primäre Antikörper gegen das p40 BDV-Nukleoprotein gerichtet ist.

Es zeigte sich, dass der Nachweis einer akuten, derzeit aktiven Infektion möglich ist, wenn der erste primäre Antikörper nur phosphoryliertes P-Protein erkennt (Bode, 2008), Die phosphorylierte Form ist im Wesentlichen nur im aktiven Zustand der Infektion nachweisbar.

In einem zweiten Aspekt richtet sich die vorliegende Erfindung auf die Verwendung eines diagnostischen Kits für einen Sandwich ELISA zum Nachweis von akuten BDV Infektionen wie in den Ansprüchen ausgeführt. Dieses diagnostische Kit umfasst einen ersten primären Antikörper und einen zweiten primären Antikörper, wobei dieser erste primäre Antikörper gegen das p24 BDV Phosphoprotein gerichtet ist und der andere primäre Antikörper gegen das p40 BDV Nukleoprotein, wobei der primäre Antikörper gegen das p24 BDV Phosphoprotein immobilisiert vorliegt. Das Kit umfasst weiterhin mindestens einen sekundären Antikörper, der mit einem Reportermolekül markiert ist, gegebenenfalls Mittel zum Immobilisieren des primären Antikörpers an eine Oberfläche, insbesondere Mittel zum Immobilisieren des primären Antikörpers gegen das p24 BDV Phosphoprotein sowie Anleitungen zur Durchführung des erfindungsgemäßen Verfahrens.

In einem dritten Aspekt richtet sich die vorliegende Erfindung darauf, akute BDV-Infektionen von chronischen und ruhenden Infektionen zu unterscheiden wie in dem Verfahren in den Ansprüchen ausgeführt. Zur Unterscheidung von chronischen Infektionen wird die vorliegende Erfindung weiterhin kombiniert mit Verfahren wie zum Beispiel in der DE 19758017 C2 beschrieben, zum Nachweis von BDV Infektionen, bei denen die frei zirkulierenden Immunkomplexe (CIC) aus BDV Antigenen und hieran angelagerten spezifischen Antikörpern durch immunologische Tests nachgewiesen werden.

Zur Unterscheidung von ruhenden Infektionen wird das erfindungsgemäße Verfahren kombiniert mit Verfahren zum Nachweis von BDV-Infektionen, bei denen frei zirkulierende BDV-Antikörper gegen das p24 Protein und/oder das p40 Protein im infizierten Wirt durch immunologische Tests nachgewiesen werden.

### Ausführliche Beschreibung der Erfindung

Die vorliegende Erfindung betrifft ein Verfahren zum Nachweis akuter Borna Disease Virus (BDV) Infektionen gemäß der Ansprüche, umfassend den Schritt des Bestimmens des Vorliegens von Heterodimeren aus p24 BDV-Phosphoprotein und p40 BDV-Nukleoprotein in einer Probe mittels eines Sandwich-ELISA, wobei ein erster primärer Antikörper immobilisiert vorliegt und ein zweiter primärer Antikörper nicht immobilisiert nach Inkubation der Probe mit dem immobilisierten ersten Antikörper hinzugefügt wird, wobei der erste primäre Antikörper gegen das p24 BDV-Phosphoprotein gerichtet ist und der zweite primäre Antikörper gegen das p40 BDV-Nukleoprotein gerichtet ist. Es zeigte sich, dass durch Verwendung der beiden genannten primären Antikörper, nämlich einerseits des Antikörpers gegen das p24 BDV Phosphoprotein und andererseits des primären Antikörpers gegen das p40 BDV Nukleoprotein, Heterodimere gebildet aus diesen beiden Proteinen nachgewiesen werden können. Solche Heterodimere, in denen das p24 in phosphorylierter Form vorliegt, liegen insbesondere in der akuten Phase vor. Dadurch ist es möglich, eine akute BDV Infektion zweifelsfrei nachzuweisen.

Der CIC-ELISA hat sich als Basistest zum Nachweis einer chronischen BDV-Infektion bewährt. Für den Nachweis der akuten BDV-Infektion, der für klinisch Erkrankte besonders wichtig ist, ist dieser Test alleine nicht geeignet. Der bisherige Antigen-Test ist eingeschränkt durch hohes Probenvolumen und limitierte Ressourcen und damit auf längere Sicht nicht als diagnostisches Kit einsetzbar.

Die vorliegende Erfindung schließt diese Lücke durch Bereitstellung eines komplett neuen Testverfahrens für akute BDV-Infektionen. Durch Kombination mit CIC-Test und Antikörpertest ist das neue Verfahren in der Lage, akute, chronische und ruhende Infektionen zu unterscheiden und die BDV-Diagnostik damit mit hohem Qualitätsniveau auf eine bisher unerreichte Aussagekraft zu heben.

Die vorliegende Erfindung stellt ein komplett neues Testverfahren für akute BDV-Infektionen bereit, weil unter anderem die Bindungsprofile der in den Verfahren DE 19758017 C2 eingesetzten monoklonalen Antikörpern "W1" und "Kfu2" entschlüsselt werden konnten, die insbesondere die hohe Spezifität des neuen Verfahrens bestimmen. Die Aminosäuresequenzen der Bindungsstellen, die die jeweiligen Konformationsepitope bilden, werden hier erstmalig benannt (Seq. ID. Nr 3 bis 7 für das p40 und Seq. ID. Nr. 8 bis 10 für p24). Sie stellen essentielle molekulare Bestandteile der Eigenschaften der o.g. monoklonalen Antikörper dar.

Die Anforderungen an die Spezifität der Primärantikörper sind dergestalt, dass sie monoklonal sind und Konformationsepitope erkennen, die nicht mit der Bindungsdomäne des BDV Phosphoproteins auf dem BDV-Nukleoprotein überlappen, damit Heterodimere erkannt werden können. Des Weiteren sind die Anforderungen der gegen das Phosphoprotein gerichteten Antikörper bevorzugt dergestalt, dass das Epitop die Hauptphosphorylierungsstelle (Schwemmle et al., 1997) einschließt und nur die aktive phosphorylierte Form des Proteins erkennt.

Erfindungsgemäß ist daher der erste primäre Antikörper gegen das phosphorylierte p24 BDV Phosphoprotein gerichtet. Hier werden erfindungsgemäß nur p24 phosphorylierte Heterodimere aus p24 BDV Phosphoprotein und p40 BDV Nukleoprotein detektiert.

Die BDV-Antigene p24 (P-Protein) und p40 (N-Protein) werden beim Aktivitätsschub vermehrt gebildet und im Körper in das Blutplasma entlassen. Diese p24 und p40 Proteine bilden dann im Blut bevorzugt Heterodimere aus p24 und p40 aus, wobei das p24 Protein in dem Heterodimer nach aktiver Virusvermehrung und Sezernierung ins Plasma phosphoryliert vorliegt. Die Phosphorylierung ist ein aktiver Prozess der Wirtszelle nach Bildung des viralen P-Proteins.

Der Nachweis einer akuten, derzeit aktiven BDV-Infektion ist insbesondere dadurch möglich, dass mit dem erfindungsgemäßen Verfahren Heterodimere mit phosphoryliertem BDV p24 Phosphoprotein nachgewiesen werden. Erfindungsgemäß ist dabei dieser gegen die phosphorylierte Form gerichtete Antikörper ein monoklonaler Antikörper, zum Beispiel ein aus der Maus stammender monoklonaler Antikörper. Dabei ist dieser Antikörper der erste primäre Antikörper, der an einer Oberfläche immobilisiert ist.

Der Nachweis einer akuten, derzeit aktiven BDV-Infektion ist dadurch gesichert, dass der die phosphorylierte Form bindende monoklonale Antikörper, wie der vorrangig verwendete mAK "Kfu2", siehe Ludwig et al., 1993, ein Konformationsepitop erkennt, das aus drei Bindungsstellen auf dem aktiven phosphorylierten BDV-P-Protein geformt wird (Seq. ID Nrn. 8, 9 und 10). Der zweite primäre monoklonale Antikörper, wie der vorrangig verwendete mAk "W1", siehe Ludwig et al., 1993, erkennt ein Konformationsepitop, das aus fünf Bindungsstellen auf dem BDV N-Protein geformt wird (Seq. ID Nrn 3, 4, 5, 6 und 7). Es besteht keine Überlappung dieser Bindungsstellen auf beiden Proteinen mit der Bindungsdomäne des P-Proteins (Berg et al., 1998) auf dem N-Protein. Dadurch können N/P Heterodimere erkannt werden (Bode, 2008).

Das Immobilisieren des ersten primären Antikörpers an die Oberfläche erfolgt in einer Ausführungsform direkt, in einer alternativen Ausführungsform erfolgt die Immobilisierung über einen ersten Sekundärantikörper, der zum Beispiel speziesspezifisch, insbesondere subklassenspezifisch den primären Antikörper bindet, ohne dass die Aktivität dieses primären Antikörpers zur Bindung an das Heterodimer aus p24 BDV Phosphoprotein und p40 BDV Nukleoprotein, insbesondere das p24 phosphorylierte BDV Phosphoprotein, beeinträchtigt wird.

Die Immobilisierung des primären Antikörpers oder des ersten Sekundärantikörpers an eine Oberfläche erfolgt gemäß üblicher Verfahren. Dem Fachmann sind entsprechende Verfahren bekannt. Die Immobilisierung erfolgt üblicherweise über die Fc-Region des Sekundärantikörpers oder des Primärantikörpers.

Das vorliegend beschriebene Verfahren zum Infektionsnachweis im Serum/Plasma basiert in erster Linie auf der Infektion peripherer mononukleärer Blutzellen (PBMCs) dergestalt, dass die in Schüben verlaufende Vermehrung des Virus zur Sezernierung von N- und P-Protein (Antigene) aus PBMCs (und noch unbekannten Quellen im Körper) ins Blutplasma führt (akute Infektion), im weiteren Verlauf Antikörper im Wirt gebildet werden und diese gebunden an die Antigene zu zirkulierenden Immunkomplexen (CIC) werden, die eine chronische Infektion anzeigen. Der Begriff "Nachweis" wird vorliegend derart verwendet, dass sowohl ein erster qualitativer Nachweis als auch eine quantitative Bestimmung der Konzentration an Heterodimeren möglich ist.

In einer weiteren Ausführungsform des erfindungsgemäßen Verfahrens wird der nicht-immobilisierte primäre zweite Antikörper über einen zweiten ein Reportermolekül aufweisenden Sekundärantikörper bestimmt.

Unter dem Ausdruck "Reportermolekül" wird vorliegend ein Molekül verstanden, das einerseits eine direkte Messung ermöglicht, zum Beispiel auch bekannt als Label oder Tag. Andererseits kann dieses Reportermolekül einen ersten Bindungsparameter aufweisen, der entsprechend mit einem weiteren markierten Molekül (zweiter Bindungspartner) als Bindungspaar nachgewiesen wird. Schließlich kann das Reportermolekül einen Katalysator umfassen, wie ein Enzym.

Beispiele für geeignete Reportermoleküle umfassen bekannte Reportermoleküle, wie Farbstoffe einschließlich Fluoreszenzfarbstoffe oder radioaktive Moleküle. Alternativ kann dieses Reportermolekül ein bekanntes Bindungspaar sein, wie ein Biotin-Streptavidin beziehungsweise Biotin-Avidin-Paar. Schließlich kann das Reportermolekül ein Katalysator insbesondere ein Enzym sein. Bekannte Enzyme schließen die alkalische Phosphatase, die Peroxidase insbesondere die Meerrettichperoxidase und andere, übliche Enzyme ein.

Dem Fachmann sind entsprechende Reportermoleküle, wie sie insbesondere in immunbasierten Nachweisverfahren eingesetzt werden, insbesondere in ELISA-Verfahren, bekannt.

In einer Ausführungsform ist der zweite Sekundärantikörper einer, der Biotin als Reportermolekül aufweist. Dieser Antikörper wird dann mithilfe eines entsprechenden Bindungspartners, wie Avidin oder Streptavidin gekoppelt mit einem Enzym, wie alkalische Phosphatase (AP) oder Meerrettichperoxidase (HRP) nachgewiesen.

Alternativ kann dieser zweite Sekundärantikörper direkt mit einem Enzym, wie der AP oder der HRP markiert sein.

In einer Ausführungsform sind dabei die beiden Sekundärantikörper von derselben Spezies, in einer anderen Ausführungsform stammen die beiden Sekundärantikörper von unterschiedlichen Spezies. In einer Ausführungsform sind die beiden Sekundärantikörper solche, die aus der gleichen Spezies oder unterschiedlichen Spezies stammend unterschiedliche Immunglobulinsubklassen der Spezies der Primärantikörper erkennen.

Die Primärantikörper können in einer Ausführungsform der vorliegenden Erfindung solche sein, wobei der erste primäre Antikörper und der zweite primäre Antikörper aus verschiedenen Spezies stammen und wobei diese primären Antikörper monoklonale Antikörper sind. In einer Ausführungsform hiervon stammen die primären Antikörper aus der gleichen Spezies aber von unterschiedlichen Immunglobulinklassen oder unterschiedlichen Immunglobulinsubklassen. In einer Ausführungsform stammen die primären Antikörper dabei aus der Maus und sind monoklonale Antikörper, zum Beispiel wobei einer der Primärantikörper einer der Subklasse IgG1 ist und der zweite primäre Antikörper ein Antikörper aus der Maus der Subklasse IgG2 ist.

Wenn die primären Antikörper unterschiedlichen Subklassen entstammen, wie IgG1 und IgG2, dann sind bevorzugt die sekundären Antikörper solche, die spezifisch diese Subklassen detektieren, das heißt an diese Subklassen binden. So kann die Kreuzreaktivität gering gehalten werden.

Die Primärantikörper können gegebenenfalls derart verändert sein, dass sie andere Komponenten aufweisen oder single-chain Antikörper sind. Die Primärantikörper zeichnen sich dadurch aus, dass sie spezifisch das entsprechende Protein des BDV erkennen und die notwendige Bindungsstelle, wenn erforderlich, für den Sekundärantikörper aufweisen. Wenn der Primärantikörper direkt an die Oberfläche gekoppelt wird, muss diese Bindungsstelle für den Sekundärantikörper nicht vorhanden sein. Bei direkter Kopplung kann der Primärantikörper derart verändert sein, dass die Bindung an die Oberfläche begünstigt wird.

Dem Fachmann sind geeignete Maßnahmen bekannt, die Primärantikörper durch entsprechende molekularbiologische Verfahren zu verändern. Dabei bleibt die Spezifität des primären und gegebenenfalls sekundären Antikörpers erhalten, während Komponenten, die gegebenenfalls eine Kreuzreaktivität verursachen, herausgenommen werden. Diese Veränderungen unterstützen die Verbesserung der Spezifität.

Der Ausdruck "Antikörper" wie er hierin verwendet wird schließt monoklonale Antikörper, polyklonale Antikörper und chimere Antikörper ein, solange nicht anders ausgeführt. Die Antikörper können von rekombinanten Quellen und/oder durch Hybridomzellen hergestellt werden. Alternativ können transgene Tiere etc. eingesetzt werden. Die Antikörper können auch als Antikörperfragmente vorliegen insoweit sie die Spezifität für das p24 BDV Phosphoprotein und p40 BDV Nukleoprotein behalten. Der Ausdruck "Antikörperfragmente", wie er hierin verwendet wird, schließt geeignete scFv, dsFv, ds-scFv, Dimere, Minibodies, Diabodies aber auch fab, fab- oder f(ab')2 Fragmente ein.

Solche Fragmente können durch entsprechende rekombinante Techniken hergestellt werden.

In der vorliegenden Erfindung wird monoklonalen Antikörpern der Vorzug gegeben, die mit geeigneten herkömmlichen Techniken, also Hybridomtechniken, hergestellt werden. Mit dieser Technik in Kombination mit Infektion und Immunisierung der Mäuse konnten Antikörper selektioniert werden, die jeweils Epitope auf der nativen Konformation der Proteine N und P erkennen. Konformationsepitope, wie sie vorliegen für "W1" und "Kfu2", weisen besonders hohe Bindungskonstanten für native BDV-Proteine auf (Bode, 2008), die für lineare Epitope von mit Hilfe rekombinanter Proteine hergestellten monoklonalen Antikörpern nicht erreicht werden.

Das Reportermolekül, auch als Marker oder Label bezeichnet, erlaubt die direkte oder indirekte Generierung von detektierbaren Signalen. Geeignete Label oder Marker sind Radioisotope, wie sie allgemein bekannt sind, insbesondere aber auch Farbstoffe, wie Fluoreszenzfarbstoffe (Fluorophore) oder Chemilumineszenzverbindungen (Chromophore), Beispiele hierfür sind Fluoreszein-Isothiocyanat (FITC), Rhodamin oder Luziferin. In einer Ausführungsform handelt es sich bei den Reportermolekülen um Enzyme insbesondere alkalische Phosphatase oder β- Galactosidase oder Meerrettichperoxidase, die Substrate mit Farbumschlag als Signal umsetzen. Bildgebende Mittel wie magnetische oder paramagnetische Markermoleküle oder Metallionen sind ebenfalls eingeschlossen. In einer Ausführungsform ist das Reportermolekül über einen Bindungspartner eines Bindungspaares wie zum Beispiel dem Streptavidin-Biotin-Bindungspaar oder Biotin-Avidin-Bindungspaar an den Sekundärantikörper gekoppelt. Dem Fachmann sind geeignete Bindungspaare bekannt.

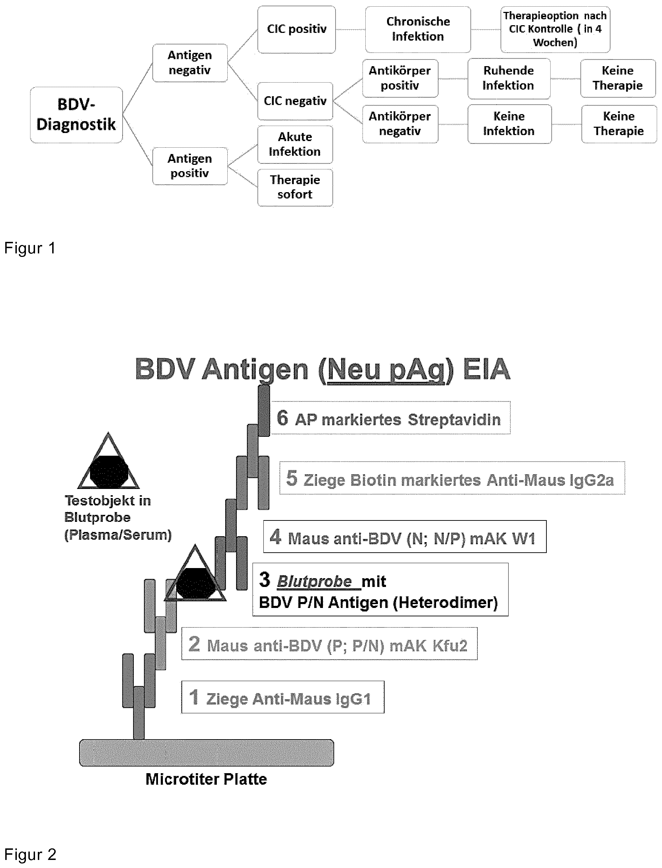

In einer weiteren dritten Ausführungsform wird neben dem Nachweis des Heterodimers weiterhin zusätzlich ein Nachweis zirkulierender Immunkomplexe (CIC) aus BDV-Antigenen und daran angelagerten spezifischen Antikörpern nachgewiesen. Entsprechende Nachweisverfahren sind zum Beispiel aus der DE 19758017 C2 bekannt. Die Kombination des Nachweises des phosphorylierten Heterodimers gemäß der vorliegenden Erfindung mit dem Nachweis zirkulierender Immunkomplexe (CIC) und darin gebundener spezifischer Antikörper aus dem Individuum erhöhen die Spezifität, eine BDV Infektion nachzuweisen, insbesondere auch die Differenzierung zwischen akuter BDV Infektion und chronischer BDV Infektion. In einer weiteren Ausführungsform wird neben dem Nachweis des Heterodimers und den CIC das auch aus der DE 19758017 C2 bekannte Nachweisverfahren für BDV-Antikörper ausgeführt. Die Kombination des Nachweises des Heterodimers gemäß der vorliegenden Erfindung mit dem CIC-Nachweis und dem Nachweis freier Antikörper ermöglicht die Differenzierung von akuter, chronischer und ruhender Infektion im Individuum, siehe auch Figur 1.

Das erfindungsgemäße Verfahren ist insbesondere eines, das die Diagnose und/oder Verlaufskontrolle ermöglicht und/oder zum Ausschluss einer BDV-Infektion z. B. bei psychiatrischen und/oder neurologischen Erkrankungen eingesetzt wird. Das heißt, das Verfahren ist erfindungsgemäß eines, das eine akute BDV Infektion diagnostiziert. In einem anderen Aspekt ist es ein Verfahren, das geeignet ist zur Verlaufskontrolle in Kombination mit CIC-Test und Antikörper-Test und insbesondere zur Differenzierung zwischen akuter, chronischer und ruhender BDV Infektion. Es ist ebenfalls geeignet, in obiger Kombination, BDV-Infektionen verschiedener Aktivitätszustände bei psychiatrischen und/oder neurologischen Erkrankungen nachzuweisen oder auszuschließen.

In einem weiteren Aspekt richtet sich die vorliegende Erfindung auf die Verwendung eines diagnostischen Kits für einen Sandwich ELISA zum Nachweis akuter BDV Infektionen wie in den Ansprüchen definiert. Dieses diagnostische Kit umfasst einen ersten primären Antikörper und einen zweiten primären Antikörper. Einer dieser beiden primären Antikörper ist gegen das p24 BDV Phosphoprotein gerichtet, der andere primäre Antikörper gegen das p40 BDV Nukleoprotein. Das diagnostische Kit umfasst weiterhin geeignete Sekundärantikörper mindestens aber einen Sekundärantikörper, der mit einem Reportermolekül markiert ist. Gegebenenfalls werden weiterhin Mittel zum Immobilisieren eines primären Antikörpers an eine Oberfläche sowie Anleitungen zur Durchführung des erfindungsgemäßen Verfahrens mit diesem Kit bereitgestellt.

Bei den im Kit vorhandenen primären Antikörpern und sekundären Antikörpern handelt es sich um solche wie vorliegend beschrieben. Die primären Antikörper sind solche, wo einer dieser primären Antikörper das phosphorylierte Protein des p24 phosphorylierten Heterodimers erkennt, während der zweite primäre Antikörper das p40 BDV Nukleoprotein im Heterodimer erkennt. Die Sekundärantikörper sind solche Sekundärantikörper wie in der vorliegenden Anmeldung definiert, insbesondere handelt es sich dabei um solche, die aus der gleichen Spezies stammen und/oder solche, die unterschiedliche Subklassen primärer Antikörper erkennen.

In einer Ausführungsform umfasst das diagnostische Kit weiterhin Mittel zum Nachweis von CIC ausgebildet aus BDV-Antigenen und daran angelagerten spezifischen Antikörpern des Individuums. Diese Mittel sind insbesondere entsprechende monoklonale Antikörper.

Schließlich wird die Verwendung des erfindungsgemäßen Verfahrens und des diagnostischen Kits zur Diagnose und/oder Verlaufskontrolle und/oder zum Ausschluss von BDV-Infektionen bei psychiatrischen und/oder neurologischen Erkrankungen bereitgestellt.

Das erfindungsgemäße Verfahren und die Verwendung des Verfahrens und des diagnostischen Kits erlauben, einen Behandlungsplan für die BDV Infektion in dem untersuchten Individuum als Option für den behandelnden Arzt aufzustellen. Entsprechend wird ein Verfahren zur Behandlung akuter BDV Infektionen bereitgestellt, umfassend das Verfahren zum Nachweis einer akuten BDV Infektion gemäß der vorliegenden Erfindung und bei Diagnose einer akuten BDV Infektion Behandlung dieser mit dem antiviralen Medikament Amantadinsulfat (AS). Dieses Medikament hat sich als hochwirksam gegen natürliche Borna-Viren erwiesen. AS ist ein seit über 40 Jahren zur antiviralen Therapie der Virusgrippe (Influenza A) zugelassenes Medikament, das zur Behandlung der Borna-Virus-Infektion verschrieben werden kann (Off-Label-Use). AS hemmt die Vermehrung natürlicher Borna-Viren und damit die Bildung der schädlichen Viruseiweiße (Antigene) (Bode und Ludwig, 2003). Die Mehrheit infizierter akut und/oder chronisch depressiver Patienten (70%) profitiert nachhaltig durch Besserung der Symptomatik (Dietrich et al., 2000; Ferszt et al., 1999) parallel zum Rückgang der Virusmarker im Blut. Die antivirale Wirkung von AS konnte auch bei infizierten Patienten gezeigt werden, deren Depression bereits in der Remission war (keine depressiven Symptome mehr). Die Verlaufskontrolle im Blutplasma mit Anwendung der ELISA-Tests zur Bestimmung der CIC, des Antigens (pAG, früherer Test) und der Antikörper nach Verfahren DE 198 60 255 C2 zeigte eine signifikante Reduktion dieser Testparameter nach 14 Wochen (Dietrich and Bode, 2008).

AS ist nachweislich wirksam bei natürlichen Borna-Viren von Mensch und Pferd *in vitro* in dosisabhängiger Weise. Der Titer des humanen Virusisolats Hu-H1 aus einer depressiven bipolaren Patientin (Bode et al., 1996) in humanen Oligodendrogliazellen ist in Abhängigkeit von Amantadinsulfat Konzentrationen von 1,2 µg/ml bis 0,4 µg/ml innerhalb von 10 bis 33 Tagen nicht mehr nachweisbar. Die Konzentration von 0,4 µg/ml AS korreliert mit der Konzentration, die bei oraler Einnahme von 200 mg AS pro Tag im Patientenplasma erreicht wird (Bode et al., 1997).

Weiterhin wird ein Verfahren zur Behandlung psychiatrischer und/oder neurologischer Erkrankungen bereitgestellt, umfassend den Nachweis einer BDV Infektion insbesondere einer akuten BDV Infektion gemäß der vorliegenden Erfindung und bei Nachweis einer BDV Infektion insbesondere Nachweis einer akuten BDV Infektion oder Nachweis einer chronisch aktivierten Infektion in Kombination mit CIC. Eine Behandlungsoption des Individuums kann eine Behandlung sein mit 2 - 4 mg AS pro kg Körpergewicht täglich oral. Das sind bei einem 75 Kg schweren Patienten 150 bis maximal 300 mg AS täglich. Einschleichen zu Beginn mit 1 mg AS pro Kg Körpergewicht für die ersten drei bis vier Tage. Einnahmeschema morgens in der Einschleichphase. Einnahmeschema danach, beginnend mit 2 mg AS pro Kg Körpergewicht, mit der Hälfte der Tagesdosis morgens und der anderen Hälfte der Tagesdosis mittags (1-1-0). Bei Unruhe in den ersten ein bis zwei Wochen oder Einschlafstörungen, die Tagesdosis nur morgens nehmen (1-0-0).

Weiterhin wird ein Verfahren zur Behandlung einer BDV Infektion bereitgestellt, wobei die Behandlung überwacht wird im Rahmen einer Verlaufskontrolle mithilfe des erfindungsgemäßen Verfahrens. Die Behandlung erfolgt dabei derart, dass bei positivem Behandlungsverlauf, gekennzeichnet durch eine klinische Besserung durchschnittlich nach den ersten vier bis sechs Wochen und eine reduzierte Virusaktivität, gekennzeichnet durch Verringerung der Heterodimere und/oder der CIC im Blutplasma, die Behandlung nach drei Monaten abgeschlossen werden kann. Eine Kontrolle der Blutwerte für Antigen (Heterodimere) und CIC ist obligatorisch nach drei Monaten. Vor Absetzen des Medikaments sollten keine Heterodimere mehr nachweisbar sein und niedrigere CIC-Werte im Vergleich zum Status vor der Behandlung. Das Medikament sollte unter Dosisverringerung über vier bis sieben Tage ausgeschlichen werden.

Bei verzögertem Behandlungsverlauf, gekennzeichnet durch geringe klinische Besserung nach den ersten vier bis sechs Wochen und nur geringer Veränderung oder unveränderter Detektion dieser Heterodimere insbesondere der phosphorylierten Heterodimere gegebenenfalls in Kombination mit CIC, sollte die Behandlung über die Dauer von drei Monaten fortgesetzt und gegebenenfalls die Therapie verändert werden. Vorsorglich ist bei geringer klinischer Besserung eine Verlaufskontrolle nach 6 Wochen mithilfe des erfindungsgemäßen Verfahrens in Kombination mit CIC obligatorisch und eine Anpassung der therapeutischen Dosis angezeigt bei wenig oder unveränderten Antigen und/oder CIC-Werten. Wenn die Dosis bisher 2 mg AS pro Kg Körpergewicht war, ist eine Erhöhung auf 3 mg AS pro Kg Körpergewicht bis höchstens 4 mg AS pro Kg Körpergewicht in der beschriebenen Konstellation angezeigt.

Übliche Therapien einer BDV-Infektion bei depressiven psychiatrischen Patienten schließen ein die Verordnung gängiger Antidepressiva, wie z.B. von trizyklischen Antidepressiva oder von Serotonin-Wiederaufnahmehemmern (SSRIs). AS wird unverändert in der Niere ausgeschieden, es gibt keine Abbauprodukte in der Leber und dadurch keine oder vernachlässigbare unerwünschte Wechselwirkungen mit diesen Medikamenten. AS kann daher ohne Absetzen der obigen Therapeutika zusätzlich zur Bekämpfung der BDV-Infektion verordnet werden. Bei dialysepflichtigen Patienten mit Niereninsuffizienz kann es zur unerwünschten Erhöhung der Blutspiegel von AS kommen. Daher wird bei solchen Patienten die Einnahme der oben genannten Dosierung nur jeden zweiten oder dritten Tag empfohlen und ggfs. eine wöchentliche Kontrolle der Blutspiegel des Medikaments.

Als Probe können Körperflüssigkeit und Körpergewebe in isolierter Form eingesetzt werden. Insbesondere geeignete Proben stammen aus dem Blut, z. B. in Form von Serum oder Plasma. Weitere geeignete Proben sind Cerebrospinalflüssigkeit (Liquor) bei neurologischen Patienten, schließen aber bei negativem Befund eine Infektion nicht aus, weswegen eine Probe aus Blut, als Serum oder Plasma, obligatorisch ist. In einer Ausführungsform ist die Probe eine isolierte Probe aus Blutplasma oder Blutserum.

### Beschreibung der Figuren

Figur 1 zeigt das Diagnostikschema für akute, chronische und ruhende BDV-Infektionen. In einem ersten Test wird gemäß dem erfindungsgemäßen Verfahren die Anwesenheit des Antigens getestet. Bei Vorliegen hiervon besteht eine akute Infektion, während bei einem negativen Nachweis weiter geprüft wird, ob CIC vorliegen oder nicht. Liegen CIC vor, wird von einer chronischen Infektion ausgegangen, bei CIC negativen Proben wird zusätzlich geprüft, ob Antikörper vorliegen. Liegen auch keine Antikörper vor, so wird das Vorliegen einer Infektion ausgeschlossen, während beim ausschließlichen Nachweis von Antikörpern (anti-p24 Protein und/oder anti-p40 Protein Antikörper) von einer ruhenden Infektion ausgegangen wird.
Figur 2 zeigt ein Schema des BDV-Antigen Enzym Immun Assay (EIA) gemäß dem erfindungsgemäßen Verfahren. Dabei wird der gegen phosphoryliertes p24-Protein gerichtete monoklonale Maus-Antikörper Kfu2 über die Bindung an einen Sekundärantiköper mit anti-Maus IgG1-Subklassenspezifität immobilisiert an eine Mikrotiterplatte und dann zum Fangen der Heterodimere aus der Plasma/Serumprobe eingesetzt. Diese gebundenen Heterodimere werden dann mit dem gegen p40-Protein gerichteten monoklonalen Maus-Antikörper W1 nachgewiesen. Zur Sichtbarmachung der Reaktion schließt sich ein Sekundärantikörper mit anti-Maus IgG2a-Subklassenspezifität an, über dessen Markierung mit Reportermolekül Biotin und nachfolgender Streptavidin vermittelter Bindung von Alkalischer Phosphatase die entsprechende Farbreaktion nach Zusatz des Substrats p-Nitrophenylphosphat erfolgen kann.

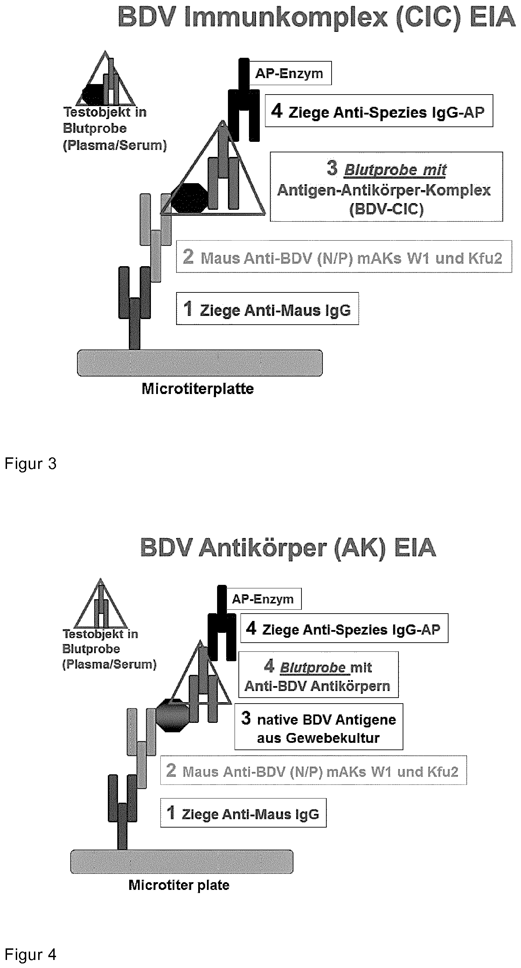

Figur 3 zeigt ein Schema des BDV-Immunkomplex EIA, wie er zum Beispiel in der DE 19758017 C2 beschrieben ist. Dort werden beide monoklonalen Maus-Antikörper W1 und Kfu2 über die Bindung an einen Sekundärantikörper mit anti-Maus IgG- Spezifität immobilisiert an eine Mikrotiterplatte und dann zum Fangen der Antigen-Antikörper-Komplexe aus der Plasma/Serumprobe eingesetzt. Diese Antigen-Antikörper-Komplexe (BDV-CIC) werden dann mit enzym- markierten Sekundärantikörpern, die gegen die IgG der Wirtsspezies der Probe gerichtet sind (Reportermolekül Alkalische Phosphatase) und einer entsprechenden Farbreaktion (wie in Figur 2) nachgewiesen.
Figur 4 zeigt ein Schema des BDV-Antikörper EIA, wie er erfindungsgemäß eingesetzt werden kann. Hierbei werden ebenfalls die beschriebenen monoklonalen Antikörper W1 und Kfu2 über die Bindung an einen Sekundärantikörper mit anti-Maus IgG-Spezifität immobilisiert an eine Mikrotiterplatte und dann als Fangantikörper eingesetzt, um zunächst native BDV-Antigene zu binden, die zuvor in virusfreier Form aus standardisierter infizierter Zellkultur gewonnen worden sind. Diese gebundenen BDV-Antigene ermöglichen dann anti-BDV-Antikörper (anti-p24 oder anti-p40 Protein Antikörper) nachzuweisen aus einer Plasma/Serumprobe. Dies erfolgt nach Bindung von in der Probe vorliegenden anti-BDV-Antikörpern an das native Antigen dann über enzym-markierte Sekundärantikörper, die gegen die IgG der Wirtsspezies der Probe gerichtet sind (Reportermolekül Alkalische Phosphatase) und eine entsprechende Farbreaktion (wie in Figur 2).

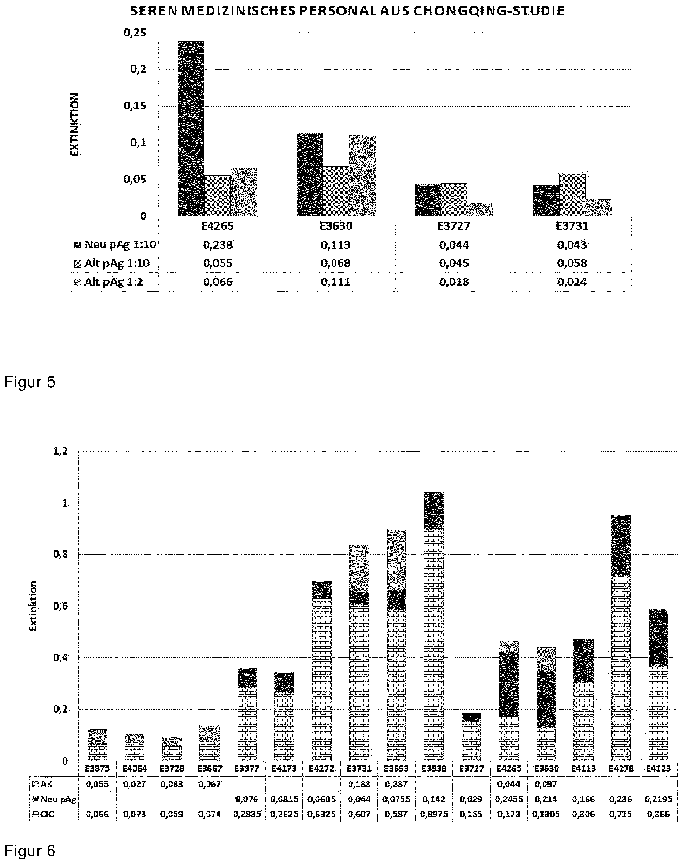

Figur 5 zeigt die Ergebnisse von Antigentests bei individuellen Proben von Testpersonen, wobei einerseits BDV-Antigen mit dem erfindungsgemäßen Verfahren (Neu pAg) und andererseits mit dem aus der DE 198 60 255 C2 beschriebenen früheren Antigennachweis-Verfahren (Alt pAg) nachgewiesen wird.
Figur 6 zeigt die Ergebnisse der kombinierten Anwendung des erfindungsgemäßen Antigentests mit dem CIC-Test und dem Antikörper Test, die beiden letzteren Tests wie in DE 19758017 C2 beschrieben. Die Extinktionswerte sind kumulativ dargestellt.

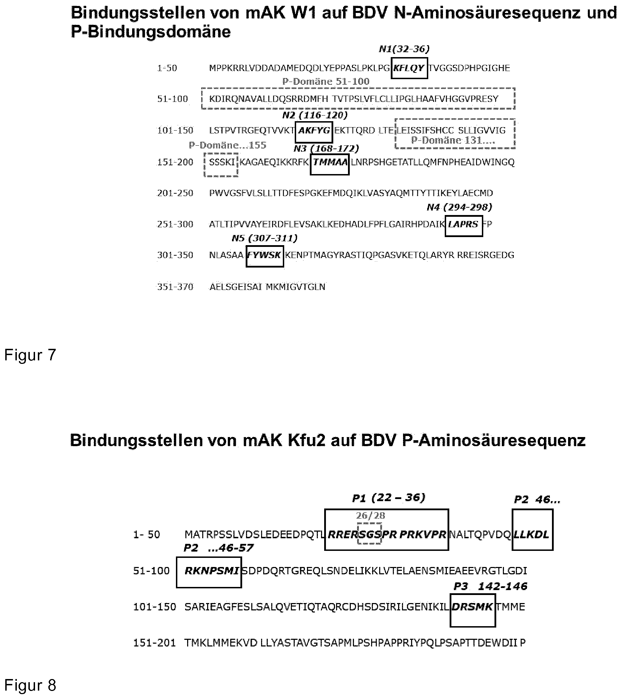

Figur 7 zeigt die vollständige Aminosäuresequenz des BDV-N-Proteins (Seq. ID. Nr. 1). Die fünf Bindungsstellen N1 bis N5 (Seq. ID Nrn. 3 bis 7) für den beschriebenen monoklonalen Antikörper W1, identifiziert mit Epitopmapping, bilden das Konformationsepitop auf dem nativen BDV-N-Protein, wobei N1, N3 und N5 interaktive Bindungsstellen sind nach Vergleich mit Kristallstruktur (Bode, 2008). Die Bindungsstellen sind schwarz eingerahmt. Gestrichelt eingerahmt sind die beiden Domänen, die die Bindungsstellen des P-Proteins auf dem N-Protein, Aminosäure 51-100 und 131-155, definieren (Berg et al., 1998). Diese zeigen keine Überlappung mit den W1-Bindungsstellen. Dadurch können auch N/P-Heterodimere erkannt werden.
Figur 8 zeigt die vollständige Aminosäuresequenz des BDV-P-Proteins (Seq. ID Nr. 2). Die drei Bindungsstellen P1 bis P3 (Seq. ID. Nrn. 8 bis 10) für den beschriebenen monoklonalen Antikörper Kfu2, identifiziert mit Epitopmapping, bilden das Konformationsepitop auf dem nativen P-Protein. Die Bindungsstellen sind schwarz eingerahmt. P1 ist die Hauptbindungsstelle und schließt die Hauptphosphorylierungsstelle (S26/S28; Schwemmle et al., 1997) auf dem BDV-P-Protein ein (gestrichelt eingerahmt). Kfu2 erkennt ausschließlich das aktive, phosphorylierte BDV P-Protein (Bode, 2008).

Die Erfindung wird mithilfe von Beispielen weiter erläutert, ohne auf diese beschränkt zu sein.

### Beispiele

Es werden isolierte Blutproben von Individuen bereitgestellt, die eine BDV-Infektion aufzeigen, wobei der Status der Infektion entweder akut, chronisch oder ruhend war bzw. keine Infektion vorlag.

| **Tabelle von medizinischem Personal Chongqing Studie** | | | | | | | |
|---|---|---|---|---|---|---|---|
| | | | | | | | |

| Code | Medizinisches Personal (MP) | Geschlecht | Alter | **Antigen Heterodimere** | **CIC** | **Antikörper** | **Status Infektion** |
|---|---|---|---|---|---|---|---|
| Probe | | | | **1:10 verdünnt** | **1:20 verdünnt** | **1:100 verdünnt** | |
| E3875 | keine Angaben | weiblich | 50 | | **0,066** | **0,055** | **keine** |
| E4064 | Verwaltung | männlich | 51 | | **0,073** | **0,027** | **keine** |
| E3728 | Ophthalmologie | weiblich | 29 | | **0,059** | **0,033** | **keine** |
| E3667 | Gastroenterologie | männlich | 28 | | **0,074** | **0,067** | **keine** |
| E3977 | Infektionsabteilung | weiblich | 48 | **0,076** | **0,2835** | | **chronisch** |
| E4173 | Brustchirurgie | weiblich | 47 | **0,0815** | **0,2625** | | **chronisch** |
| E4272 | OP-Vorbereitung | weiblich | 49 | **0,0605** | **0,6325** | | **chronisch** |
| E3731 | Ophthalmologie | weiblich | 52 | **0,044** | **0,607** | **0,183** | **chronisch** |
| E3693 | Herzchirurgie | weiblich | 59 | **0,0755** | **0,587** | **0,237** | **chronisch** |
| E3838 | keine Angaben | weiblich | 26 | **0,142** | **0,8975** | | **akut** |
| E3727 | Ophthalmologie | weiblich | 55 | **0,029** | **0,155** | | **chronisch** |
| E4265 | Exp. Forschung | weiblich | 41 | **0,2455** | **0,173** | **0,044** | **akut** |
| E3630 | außer Dienst | weiblich | 35 | **0,214** | **0,1305** | **0,097** | **akut** |
| E4113 | Rehabilitation | männlich | 81 | **0,166** | **0,306** | | **akut** |
| E4278 | OP-Vorbereitung | männlich | 29 | **0,236** | **0,715** | | **akut** |
| E4123 | Rehabilitation | männlich | 30 | **0,2195** | **0,366** | | **akut** |
| E3968 | Positivkontrolle MP | männlich | 57 | **1,9835** | **0,3675** | **0,3025** | **akut** |
| Student | Negativkontrolle | | | | **0,0495** | **0,068** | **keine** |
| | | | | | | | |
| **Bewertung der Testergebnisse (Standard für alle BDV-EIA Teste)** | | | | | | | |
| Cut-off=0.100. **Negativ** <=0.1; **Grenzwertig** > 0.1-0.12; **(+) schwach positiv** > 0.12-0.15; **1+ positiv** > 0.15-0.3 | | | | | | | |
| **2+ positiv** > 0.3-0.6; **3+ positiv** >0.6-1.0; **4+ positiv** >1.0. | | | | | | | |

Die Proben entstammen einer großen Studie, die in einem Universitätshospital in Chongqing, China, durchgeführt worden ist unter Verwendung des CIC- und zusätzlich des Antikörpertests und einer Realtime RT-PCR (Liu et al. 2015). Die Teste wurden mit den in der DE 197 58 017 C2 verwendeten Antikörpern W1 und Kfu 2 durchgeführt. W1 stellt dabei den Primärantikörper, gerichtet gegen das P40 BDV Nukleoprotein dar, Kfu 2 stellt den Primärantikörper, gerichtet gegen phosphoryliertes P-Protein P24 Phosphoprotein von BDV dar. Der Testaufbau für den CIC-Nachweis und den Antikörper-Nachweis folgt dem jeweiligen Schema in den Figuren 3 und 4. Der Testaufbau des erfindungsgemäßen Verfahrens zum Antigennachweis (Heterodimere) folgt dem Schema in Figur 2.

### Material erfindungsgemäßes Verfahren:

AffiniPure Ziege-Anti-Maus IgG1, Fc-Fragment spezifische Antikörper (Konzentration 1,3 mg Antikörper pro ml), minimale Kreuzreaktion mit Serumproteinen vom Menschen, Rindern und Kaninchen (Code Nummer 115-005-205, Jackson ImmunoResearch).
Biotin-SP konjugierte AffiniPure Ziege-Anti-Maus IgG2a, Fc-Fragment spezifische Antikörper, minimale Kreuzreaktion mit Serumproteinen vom Menschen, Rindern und Kaninchen (Code Nummer 115-065-206, Jackson ImmunoResearch).
Alkalische Phosphatase konjugiertes Streptavidin (Code Nummer 016-050-084, Jackson ImmunoResearch).
Alkalische Phosphatase Substrat Kit (Code Nummer 172-1063, Biorad) oder Einzelsubstanzen für Substratpuffer und Substrat (Bode et al., 2001) wie folgt:
   Substratpuffer, Diethanolaminpuffer pH 9,8 (Diethanolamin 1,007 M + MgCl₂· 6 H₂O 0,5 mM + NaN₃ 0,003 M);
   Substrat, p-Nitrophenylphosphat Hexahydrat (pNPP · 6 H₂0) (0,003 M); 5 mg Tabletten (Code Nummer N9389 Sigma Aldrich); im Test immer frisch ansetzen 1 mg pNPP pro ml Substratpuffer.
   Konjugatpuffer (Alkalische Phosphatase) TBS-Tween pH 8,0 (TRIS 0,02 M + NaCl 0,14 M + KCl 0,003 M + 0,05% Tween 20).
   Waschpuffer, NaCl-Tween (0,9% NaCl + 0,05% Tween 20 + NaN3 0,003 M). Proben-Verdünnungspuffer (auch für Schritte 2, 4 und 5 in Figur 2),
   PBS-Tween pH 7,2 (KH₂PO₄ 0,0015 M + Na₂HPO₄ · 12 H₂O 0,008 M + NaCl 0,14 M + KCl 0,003 M + 0,05% Tween 20 + NaN₃ 0,003 M).
   Beschichtungspuffer, 0,01 M Natriumphosphat/0,25 M NaCl pH 7,6 (NaH₂PO₄ · 2 H₂O 0,02 M [Stock A], Na₂HPO₄ 0,02 M [Stock B]; Beschichtungspuffer enthält 65 ml Stock A + 435 ml Stock B + 0,25 M NaCl in 1000 ml Aqua bidest.

### Stoplösung, 3 M NaOH

Alle chemischen Substanzen mit Reinheitsgrad nach Spezifikation der American Chemical Society (ACS grade), vorzugsweise von Sigma Aldrich.
Nunc-Immuno 96-Loch-Mikrotiter-Platten für ELISA, MaxiSorp, F (Flachboden) (Code Nummer 442404, Nunc).

### BDV-spezifische Schlüsselreagenzien

W1 (Maus Subklasse IgG2a) spezifisch gerichtet gegen das nukleäre BDV-Protein (N-Protein);
Kfu2 (Maus Subklasse IgG1) spezifisch gerichtet gegen das BDV Phosphoprotein (P-Protein);
W1 und Kfu2 sind monoklonale Maus-Antikörper, Erstbeschreibung Ludwig et al., 1993.
Die Erfinder verfügen über bereits produzierte Hybridomüberstände dieser beiden Antikörper, die für die Testkit-Produktion im Industriemaßstab geeignet sind. Die Erfinder verfügen außerdem über Hybridomzellen, die eine unbegrenzte Herstellung weiterer Chargen der monoklonalen Antikörper W1 und Kfu2 erlauben.

### Weitere Charakterisierung von W1 und Kfu2 (Bode, 2008)

| **Kurzbezeichnung** | **W1** |
|---|---|
| Spezies | Maus |
| Subklon | TL9 |
| IgG Subklasse | IgG 2a |
| Spezifität | N-Protein p40 von BDV (Referenzstamm V) |
| **Dissoziationskonstante** (K_{D}) | 2.31×10⁻⁹ +- 0.26×10⁻⁹ M (natives N-Protein) |
| Epitop-Typ | Konformationsepitop (diskontinuierlich) |
| Hybridoma Überstand (=ready-to-use) | 1:500 Gebrauchsverdünnung in BDV-ELISAs |
| Erkennung von rekombinantem (r)N-Protein | Ja (Test mit früherem Antigen-ELISA) |
| Nachweisgrenze von gereinigtem rN | 1,5 - 3 Nanogramm pro mL |

| **Kurzbezeichnung** | **Kfu2** |
|---|---|
| Spezies | Maus |
| Subklon | 57-4-33 |
| IgG Subklasse | IgG 1 |
| Spezifität | P-Protein p24 von BDV (Referenzstamm V) |
| **Dissoziationskonstante** (K_{D}) | 3.33×10⁻⁹ +- 0.34×10⁻⁹ M (natives P-Protein) |
| Epitop-Typ | Konformationsepitop (diskontinuierlich) |
| Hybridoma Überstand (=ready-to-use) | 1:500 Gebrauchsverdünnung in BDV-ELISAs |
| Erkennung von rekombinantem (r)P-Protein nicht-phosphoryliert | Nein (Test mit früherem Antigen-ELISA) |
| Erkennung von rP, phosphoryliert *in vitro* | Ja (Test mit früherem Antigen-ELISA) |

### Erstbeschreibung der Epitopsequenzen von W1 und Kfu2

| **Epitop auf N-Protein p40 von BDV** | **SEQ ID Nr.** | **W1** |
|---|---|---|
| Epitop-Typ | | Konformationsepitop (diskontinuierlich) |
| Anzahl Bindungsstellen | | Fünf auf jeweils 5mer Peptiden (N1-N5) |
| N1 | 3 | Aminosäuresequenz 32_KFLQY_36 |
| N2 | 4 | Aminosäuresequenz 116_AKFYG_120 |
| N3 | 5 | Aminosäuresequenz 168 TMMAA 172 |
| N4 | 6 | Aminosäuresequenz 294 LAPRS 298 |
| N5 | 7 | Aminosäuresequenz 307_FYWSK_311 |

### Siehe auch Figur 7

| **Epitop auf P-Protein p24 von BDV** | **SEQ ID Nr.** | **Kfu2** |
|---|---|---|
| Epitop-Typ | | Konformationsepitop (diskontinuierlich) |
| Anzahl Bindungsstellen | | Drei (15mer, 12mer, 5mer Peptide), P1-P3 |
| P1, Hauptdomäne, Phosphorylierungsstelle Serin 26/28 | 8 | Aminosäuresequenz |
| | | 22_RRERSGSPRPRKVPR-36 |
| P2 | 9 | Aminosäuresequenz |
| | | 46_LLKDLRKNPSMI_57 |
| P3 | 10 | Aminosäuresequenz |
| | | 142_DRSMK_146 |

### Siehe auch Figur 8

### Protokoll erfindungsgemäßes Verfahren:

Je 100 µl einer 1:1000 Verdünnung des Ziege-Anti-Maus IgG1 Sekundärantikörpers verdünnt mit Beschichtungspuffer werden in die MaxiSorp F Mikrotiterplatten pipettiert und für eine Stunde bei 37° inkubiert. Anschließend werden drei Waschzyklen mit dem Waschpuffer (120 ml total) durchgeführt. Danach wird der monoklonale Antikörper Kfu2 in einer Verdünnung von 1:500 (Hybridomüberstand) in PBS- Tween mit 100 µl pro Vertiefung für eine Stunde bei 37°C inkubiert, gefolgt von drei Waschzyklen mit dem Waschpuffer. Die Proben (1:10 verdünnt mit PBS-Tween) werden mit je 100 µl pro Vertiefung in die Mikrotiterplatte pipettiert und für eine Stunde bei 37°C inkubiert, gefolgt von drei Waschzyklen mit Waschpuffer. Anschließend wird der monoklonale Antikörper W1 in einer Verdünnung von 1:500 als Hybridomüberstand verdünnt in PBS-Tween mit 100 µl pro Vertiefung hinzugefügt, für eine Stunde inkubiert bei 37°C, gefolgt von drei Waschzyklen. Anschließend wird der Ziege Anti-Maus IgG2a Sekundärantikörper markiert mit Biotin in einer Verdünnung von 1:1000 in PBS-Tween in einer Menge von 100 µl pro Vertiefung hinzugefügt, für eine Stunde bei 37°C inkubiert und mit drei Waschzyklen anschließend gewaschen.

Dann wird das alkalische Phosphatase markierte Streptavidin, verdünnt 1:1000 in Konjugat Puffer TBS-Tween pH 8,0, in einer Menge von 100 µl pro Vertiefung hinzugefügt und für 30 min bei 37°C inkubiert. Hieran schließen sich wieder drei Waschzyklen mit Waschpuffer an.

Anschließend erfolgt die Nachweisreaktion mit p-Nitrophenylphosphat (1 mg/ml) p-NPP in Substratpuffer pH 9,8. Je 100 µl der entsprechenden p-NPP-Lösung werden hinzugegeben, gefolgt von einer Inkubation bei Raumtemperatur für 2 - 5 Minuten. Achtung, eine kontinuierliche visuelle Kontrolle ist notwendig, die Negativkontrolle muss farblos bleiben, während sich die Positivkontrolle färbt. Die Farbreaktion wird durch Hinzufügen der Stopplösung (3 M NaOH) mit je 50 µl pro Vertiefung gestoppt. Die Extinktion wird sofort bei 405 nm mit einem geeigneten Multikanalphotometer bestimmt. "Blank"-Messung gegen nicht umgesetztes Substrat.

### Bestimmung der CIC gemäß den in der DE 19758017 C2 beschriebenen Verfahren

Die Bestimmung der CIC in den Proben erfolgte gemäß den in der DE 19758017 C2 beschriebenen Verfahren (siehe obiges Schema in Figur 3). Zur Optimierung des diagnostischen Kits für die CIC-Bestimmung für die Praxis wurden die Inkubationszeiten und Temperaturen vereinheitlicht auf jeweils 1 h bei 37°C für alle Schritte, außer für die Visualisierungsschritte, d.h. die Substratinkubation (C3), Stoplösungszugabe (C4) und Messung (C5). Bezüglich der Kontrolle der Entwicklung der Farbreaktion wurde so verfahren wie oben für die hier beschriebene Erfindung. Die Initialverdünnung der zu testenden Proben von 1:20 in Einzelbestimmung mit Validierung an Positiv- und Negativkontrolle bietet die gleiche Sicherheit wie die Einbeziehung weiterer Verdünnungen.

Bei der Beschreibung der Materialien werden nachfolgend die Code-Nummern ergänzt:
AffiniPure Ziege anti-Maus IgG, Fc-Fragment spezifische Antikörper (Code-Nr. 115-005-071; Jackson ImmunoResearch).
Alkalische Phosphatase-konjugierte AffiniPure Ziege anti-Human IgG, Fc-Fragment spezifische Antikörper, minimale Kreuzreaktion mit Serumproteinen vom Rindern, Pferden und Mäusen (Code-Nr. 109-055-098; Jackson ImmunoResearch) für Humanproben.
Alkalische Phosphatase-konjugierte AffiniPure Ziege anti-Pferd IgG, Fc-Fragment spezifische Antikörper (Code-Nr. 108-055-008; Jackson ImmunoResearch) für Pferdeproben.
Schlüsselreagenzien W1 und Kfu2, Substratkit oder Substratpuffer und Substrattabletten, Puffer und Nunc MaxiSorp F ELISA-Platten wie oben für die neue Erfindung beschrieben.

### Bestimmung der Antikörper in Plasma/Serum gemäß den in der DE 19758017 C2 beschriebenen Verfahren

Die Bestimmung der Antikörper gegen das p24 Protein und/oder p40 Protein in Plasma/Serum-Proben erfolgte im Grundsatz gemäß dem in der DE 19758017 C2 beschriebenen Verfahren (siehe obiges Schema in Figur 4). Analog zum CIC-Test wurden zur Optimierung des diagnostischen Kits für die Antikörper-Bestimmung für die Praxis die Inkubationszeiten und Temperaturen vereinheitlicht auf jeweils 1 h bei 37°C für alle Schritte, außer für die Visualisierungsschritte. Des Weiteren wurde anstelle der beschriebenen Initialverdünnung der Proben von 1:50 die Verdünnung auf 1:100 angepasst. Es zeigte sich, dass die Initialverdünnung der auf Antikörper zu testenden Proben von 1:100 in Einzelbestimmung mit Validierung an Positiv- und Negativkontrolle die gleiche Sicherheit bietet wie die Einbeziehung weiterer Verdünnungen.

Zur Durchführung des in der DE 19758017 C2 beschriebenen BDV-Antikörper-EIA wird Detektions-Antigen aus BDV-infizierten Zellen benötigt. Die Gebrauchsverdünnung der Detektions-Antigene aus infizierten Zellkulturen liegt je nach Charge zwischen 1:100 und 1:300.

Unter der Nr. DSM ACC2334 wurde am 12.12.1997 bei der Deutschen Sammlung für Mikroorganismen und Zellkulturen (DSMZ) in Braunschweig eine persistent mit BDV-Stamm V (Briese et al., 1994) infizierte menschliche Oligodendroglia-Zell-Linie (OLIGO/TL) hinterlegt, die die unbegrenzte Herstellung von Detektions-Antigen ermöglicht.

Für die Beschreibung der übrigen Materialien, siehe CIC-Test.

### Ergebnisse:

In der Figur 5 ist ein Vergleich des erfindungsgemäßen Verfahrens gegenüber dem bisherigen Antigentest dargestellt. Da der bisherige Antigentest mit einer Probenverdünnung von 1:2 arbeitet, werden dessen Ergebnisse sowohl für diese Verdünnung als auch für die für den erfindungsgemäßen Test geltende Verdünnung von 1:10 einbezogen.

Die Figur 5 zeigt, dass die Probe E4265 nur mit dem erfindungsgemäßen Test positiv war, während sie mit dem bisherigen Antigentest nicht erkannt wurde, weder bei 1:10 noch bei 1:2 Probenverdünnung. Die Probe E3630 war mit dem erfindungsgemäßen Test schwach positiv. Das positive Signal wurde in einer weiteren Untersuchung bestätigt (Tabelle und Figur 6). Der bisherige Antigentest zeigte zwar ebenfalls ein schwach positives bis grenzwertig positives Signal, allerdings nur bei der Verdünnung 1:2. Die nächste Verdünnungsstufe 1:4 war bereits deutlich negativ mit Extinktionswert 0,034 (nicht abgebildet), was zu einer negativen Bewertung führte. Eine Wiederholung war aufgrund des hohen Probenvolumens für eine Verdünnungsreihe ab 1:2, d.h. 100 µl, nicht mehr möglich.

Die Proben E3727 und E3731 waren negativ in beiden Antigentesten, beim bisherigen Antigentest auch bei der Verdünnung von 1:2. Beide Proben stammen vom Ärztinnen/Schwestern-Team in der Augenheilkunde der Klinik. Die Kombination mit dem CIC-Test zeigt aber, dass beide chronisch mit BDV infiziert waren (siehe Tabelle). Team-Mitglied E3731 hatte hohe CIC-Werte und zusätzlich Antikörper, was für einen kürzlich abgelaufenen akuten Virusschub spricht.

Für die Proben E4265 und E3630, die nur mit dem neuen Test bei Probenverdünnung 1:10 positiv waren, ist hervorzuheben, dass der bisherige Test auch bei seiner standardmäßig fünffach geringeren Probenverdünnung von 1:2 nicht bzw. nicht eindeutig in der Lage war, BDV-Antigene zu detektieren. Der erfindungsgemäße Test wies also mindestens eine fünffach höhere Empfindlichkeit als der bisherige Test auf und konnte nachweisen, dass die ärztlichen Mitarbeiter (E4265 weiblich, Forschung; E3630 weiblich, außer Dienst) zum Zeitpunkt der Probennahme eine akute BDV-Infektion durchmachten. Wäre nur der bisherige Antigentest verfügbar gewesen, hätte die Diagnose "chronische Infektion" gestellt werden müssen auf Grund des gleichzeitigen Nachweises von CIC, und die akute Belastung wäre übersehen worden (siehe Tabelle).

Ergebnisse der kombinierten Anwendung des erfindungsgemäßen Antigentests mit dem CIC-Test und dem Antikörper (AK)-test gemäß DE 19758017 C2 zeigt Figur 6. Die Werte aus Tabelle 1 werden so anschaulich durch kumulative Säulen dargestellt.

Die Figur 6 verdeutlicht, dass der erfindungsgemäße Test in dem Beispielpanel sechs akute Infektionen identifizieren konnte (E4123, E 4278, E4113, E3630, E 4265 und E3838). Negative Ergebnisse beim erfindungsgemäßen Test konnten in Kombination mit dem CIC-Test sechs chronische Infektionen identifizieren (E3727, E3693, E3731, E4272, E4173, E3977). Negative CIC- und Antikörperteste konnten in vier Fällen aus dem Panel eine BDV-Infektion ausschließen (E3667, E3728, E4064, E3875).

Das Probenpanel von medizinischem Personal, bei dem nach strukturiertem Interview keine mentalen Erkrankungen vorlagen, weder früher noch aktuell, wurde gewählt, um zu zeigen, dass der erfindungsgemäße Test, allein und in Kombination mit CIC- und Antikörpertest, unabhängig von klinischer Symptomatik in der Lage ist, akute, chronische und ruhende Infektionen zu differenzieren bzw. eine BDV-Infektion auszuschließen mit Untersuchung einer einzigen Blutprobe. Chronische Stressbelastungen sind häufig im medizinischen Alltag, so dass ein erhöhtes Risiko für Aktivierungen nicht ungewöhnlich erscheint. Dazu kommt ein erhöhtes Infektionsrisiko in der täglichen Versorgung kranker Menschen.

Das im Vergleich zum erfindungsgemäßen Antigentest deutlich höhere Probenvolumen von 100 µl für den bisherigen Antigentest, mit initialer 1:2 Verdünnung und anschließender Verdünnungsreihe, stand nur für punktuelle Paralleltestungen, wie in Figur 5 gezeigt, zur Verfügung für die bereits vielfach anderweitig untersuchten Studienproben.

Die Vorteile des erfindungsgemäßen neuen Verfahrens zum Antigennachweis sind auch ohne Direktvergleich vielfältig und praxisnah.

Einerseits ist eine breite Anwendbarkeit für verschiedenste Probenmaterialien möglich, das heißt für jede Art von Körper- oder Gewebeflüssigkeit. Die Messung der BDV-Aktivität erfolgt unabhängig von der Wirtsspezies. Darüber hinaus erlaubt die Bestimmung der phosphorylierten Heterodimere aus p24 und p40 die Verifizierung akuter BDV-Infektionen, da der primäre Antikörper Kfu2 nur die aktive phosphorylierte Form des BDV P-Proteins bindet. Dies ist für den hier eingesetzten monoklonalen Antikörper Kfu2 weltweit ein Alleinstellungsmerkmal.

Im Gegensatz zu dem in der DE 19758017 C2 beschriebenen Test wird vorliegend das Heterodimer bestimmt. Im Gegensatz dazu basiert das bisherige Verfahren darauf, dass zwei Fangantikörper, das heißt immobilisierte Primärantikörper gegen sowohl p24 als auch p40 eingesetzt werden, um auch monomere Formen hiervon zu bestimmen. Entsprechend konnte aber eine Differenzierung der vorliegenden phosphorylierten Heterodimere damit nicht erfolgen. Das erfindungsgemäße Verfahren nutzt in einer Ausführungsform dabei Sekundärantikörper, die eine hohe Reproduzierbarkeit erlauben. In der Ausführungsform mit dem Bindungspaar Biotin-Streptavidin gekoppelt an ein Enzym wie alkalische Phosphatase wird eine Signalverstärkung und damit verbesserte Detektierbarkeit erzielt als mit der bisherigen Sichtbarmachung des Signals allein über enzymmarkierte Anti-Antikörper. Darüber hinaus wird signifikant weniger Patientenprobe (10 µl bei Einmalbestimmung, 20 µl bei Verdünnungsreihe ab 1:10) benötigt, und die Sensitivität des Verfahrens ist höher gemäß der fünffach höheren Initialverdünnung der Proben von 1:10. Dies zeigte sich insbesondere dadurch, dass in mehr Individuen als mit dem bisherigen Antigentest eine akute BDV-Infektion identifiziert werden konnte.

Die vorliegende Erfindung stellt ein komplett neues Testverfahren für akute BDV-Infektionen bereit, das eine höhere Sensitivität aufweist als der bisherige Antigentest und unabhängig von limitierten Ressourcen ist, wie sie z.B. Kaninchen-Antikörper darstellen. Durch Kombination mit CIC-Test und Antikörpertest ist das neue Verfahren in der Lage, akute, chronische und ruhende Infektionen (Mensch und Tiere) sicher zu unterscheiden und die BDV-Diagnostik damit mit hohem Qualitätsniveau auf eine bisher unerreichte Aussagekraft zu heben.

Das erfindungsgemäße Verfahren eignet sich daher insbesondere zur Diagnose aber auch zur Verlaufskontrolle einer BDV Infektion insbesondere auch zur Bestimmung des Therapieplans. Darüber hinaus kann das erfindungsgemäße Verfahren Teil eines Behandlungsverfahrens sein.

Die bislang unveröffentlichten Aminosäuresequenzen der Bindungsstellen, die die jeweiligen Konformationsepitope bilden, werden hier erstmalig benannt. Sie stellen essentielle molekulare Bestandteile der Eigenschaften der monoklonalen Antikörper W1 und Kfu2 dar.

Die vorliegende Erfindung stellt ein komplett neues Testverfahrens für akute BDV-Infektionen bereit, das unabhängig von limitierten Ressourcen ist und speziesübergreifend bei Menschen und Tieren eingesetzt werden kann. Durch Kombination des neuen diagnostischen Kits mit dem Einsatz bekannter Verfahren bzw. Kits zum Nachweis von zirkulierenden Immunkomplexen (CIC) und Antikörpern im Plasma oder Serum ist das neue Verfahren in der Lage, akute, chronische und ruhende Infektionen (Mensch und Tiere) sicher zu unterscheiden und eine bisher unerreichte Aussagekraft in der BDV-Diagnostik zu erreichen.

### Zitierte Literatur (alphabetisch)

Berg M, et al., J Gen Virol. 1998; 79 (Pt 12):2957-2963.
Bode L. Human Bornavirus infection - towards a valid diagnostic system. APMIS Suppl 124, 2008; 116: 21-39.
Bode L, et al. The Lancet 1997; 349: 178-179.
Bode L, et al. Mol Psychiatry 1996; 1: 200-212.
Bode L, Ludwig H. Borna disease virus infection, a human mental-health risk. Clin Microbiol Rev. 2003; 16(3):534-545. Review.
Bode L, et al. Mol Psychiatry 2001; 6: 481-491.
Briese T, et al. Proc Natl Acad Sci U S A. 1994; 91(10):4362-4366.
Cubitt B, et al. J Virol. 1994; 68(3):1382-1396.
Dietrich DE, Bode L. Human Borna disease virus infection and its therapy in affective disorders. APMIS Suppl 124, 2008; 116: 61-65.
Dietrich DE, et al. Bipolar Disorder 2000; 2: 65-70.
Ferszt R, et al. Pharmacopsychiatry 1999; 32 (4):142-147
Liu X, et al. Virol J. 2015; 12: 39.
Ludwig H, et al. Arch Virol 1993; Suppl 7:111-133.
Rudolph M, et al. Structure 2003;11: 1219-1226.
Schwemmle M, et al. J Biol Chem. 1997; 272(35):21818-21823.

## Patentansprüche

1. Verfahren zum Nachweis akuter Borna Disease Virus (BDV) Infektionen, umfassend den Schritt des Bestimmens des Vorliegens von Heterodimeren aus p24 BDV-Phosphoprotein und p40 BDV-Nukleoprotein in einer Probe mittels eines Sandwich-ELISA, wobei ein erster primärer Antikörper immobilisiert vorliegt und ein zweiter primärer Antikörper nicht immobilisiert nach Inkubation der Probe mit dem immobilisierten ersten Antikörper hinzugefügt wird und diese primären Antikörper monoklonale Antikörper sind, **dadurch gekennzeichnet, dass** der erste primäre Antikörper gegen das phosphorylierte p24-BDV-Phosphoprotein gerichtet ist und der zweite primäre Antikörper gegen p40 BDV Nukleoprotein Epitope gerichtet ist, solche die nicht mit der Bindungsdomäne des BDV Phosphoproteins auf dem BDV-Nukleoprotein überlappen, zur Bestimmung von p24 phosphorylierten Heterodimeren aus p24 BDV-Phosphoprotein und p40 BDV-Nukleoprotein.

2. Verfahren nach einem der vorherigen Ansprüche, **dadurch gekennzeichnet, dass** der immobilisierte primäre Antikörper über einen ersten Sekundärantikörper an eine Oberfläche immobilisiert vorliegt oder direkt an der Oberfläche immobilisiert ist.

3. Verfahren nach einem der vorherigen Ansprüche, **dadurch gekennzeichnet, dass** der nicht immobilisierte primäre zweite Antikörper über einen zweiten ein Reportermolekül aufweisenden Sekundärantikörper bestimmt wird.

4. Verfahren nach einem der vorherigen Ansprüche, wobei der erste primäre Antikörper und der zweite primäre Antikörper aus verschiedenen Spezies stammen.

5. Verfahren nach einem der vorherigen Ansprüche, wobei die sekundären Antikörper von unterschiedlichen Spezies sind oder aus gleichen Spezies stammen.

6. Verfahren nach einem der vorherigen Ansprüche, **dadurch gekennzeichnet, dass** zusätzlich zirkulierende Immunkomplexe (CIC) aus BDV-Antigenen, insbesondere von p24 Protein und/oder p40 Protein und daran angelagerten spezifischen Antikörpern nachgewiesen werden.

7. Verfahren nach einem der vorherigen Ansprüche, **dadurch gekennzeichnet, dass** zusätzlich zirkulierende freie Antikörper gegen p24 Protein und/oder das p40 Protein, insbesondere gegen phosphoryliertes p24 Protein und gegen p40 Protein nachgewiesen werden.

8. Verfahren nach einem der vorherigen Ansprüche, **dadurch gekennzeichnet, dass** dieses Verfahren zur Diagnose und/oder Verlaufskontrolle eingesetzt wird, insbesondere zur Differenzierung akuter BDV-Infektionen von chronischen und ruhenden Infektionen und/oder deren Verlaufskontrolle und zum Ausschluss einer BDV-Infektion insbesondere bei psychiatrischen und/oder neurologischen Erkrankungen, sowie zur Bestimmung der Infektionsprävalenz bei epidemiologischen Untersuchungen zur mentalen und physischen Gesundheit der Bevölkerung, insbesondere, dass dieses Verfahren, einschließlich der Differenzierung akuter BDV-Infektionen von chronischen und ruhenden Infektionen und/oder deren Verlaufskontrolle, zur Differentialdiagnostik und zum Ausschluss einer BDV-Infektion eingesetzt wird bei anderen chronischen Erkrankungen und chronischen Infektionen mit anderen Erregern, die mit herabgesetzter Immunabwehr einhergehen, sowie bei Erkrankungen, die eine Behandlung mit Medikamenten zur Herabsetzung oder Unterdrückung des Immunsystems erforderlich machen.

9. Verwendung eines diagnostischen Kits für einen Sandwich-ELISA zum Nachweis von akuten BDV-Infektionen gemäß eines Verfahrens nach einem der Ansprüche 1 bis 8, umfassend einen ersten primären Antikörper und einen zweiten primären Antikörper, wobei einer dieser primären Antikörper gegen das p24 BDV-Phosphoprotein gerichtet ist und der andere primäre Antikörper gegen das p40 BDV-Nukleoprotein; mindestens einen sekundären Antikörper, der mit einem Reportermolekül markiert ist; gegebenenfalls Mittel zum Immobilisieren eines primären Antikörpers an eine Oberfläche sowie Anleitungen zur Durchführung eines Verfahrens nach einem der Ansprüche 1 bis 8, wobei diese primären Antikörper monoklonale Antikörper sind und wobei der erste primäre Antikörper gegen das phosphorylierte p24-BDV-Phosphoprotein gerichtet ist und der zweite primäre Antikörper gegen p40 BDV Nukleoprotein Epitope gerichtet ist, solche die nicht mit der Bindungsdomäne des BDV Phosphoproteins auf dem BDV-Nukleoprotein überlappen, zur Bestimmung von p24 phosphorylierten Heterodimeren aus p24 BDV-Phosphoprotein und p40 BDV-Nukleoprotein.

10. Die Verwendung eines diagnostischen Kits für einen Sandwich-ELISA zum Nachweis von akuten BDV-Infektionen nach Anspruch 9, wobei die primären Antikörper wie in Anspruch 1 oder 4 definiert sind und/oder wobei die sekundären Antikörper wie in einem der Ansprüche 3 und 5 definiert sind.

11. Verwendung eines diagnostischen Kits nach einem der Ansprüche 9 oder 10, weiter umfassend Mittel zum Nachweis von CIC aus BDV-Antigenen und daran angelagerten spezifischen Antikörpern.

12. Verwendung eines diagnostischen Kits nach einem der Ansprüche 9 bis 11, weiter umfassend Mittel zum Nachweis von zirkulierenden freien Antikörpern gegen das p24 Protein und/oder p40 Protein, insbesondere das phosphorylierte p24 Protein und das p40 Protein im Plasma/Serum.

13. Verwendung des Verfahrens nach einem der Ansprüche 1 bis 8 oder des diagnostischen Kits nach einem der Ansprüche 9 bis 12 zur Diagnose und/oder Verlaufskontrolle und/oder zum Ausschluss von BDV-Infektionen insbesondere bei psychiatrischen und/oder neurologischen Erkrankungen, zur Differentialdiagnostik chronischer Erkrankungen und/oder chronischen Infektionen anderer Genese, die mit einer geschwächten Immunabwehr einhergehen und/oder Erkrankungen, bei denen eine medikamentös geschwächte/unterdrückte Immunabwehr vorliegt oder zur Bestimmung der Infektionsprävalenz in der gesunden Bevölkerung.

## Claims

1. A method for detecting acute Borna disease virus (BDV) infections, comprising the step of determining the presence of heterodimers of p24 BDV phosphoprotein and p40 BDV nucleoprotein in a sample using a sandwich ELISA, wherein a first primary antibody is present immobilized and a second primary antibody is added non-immobilized after incubation of the sample with the immobilized first antibody and these primary antibodies are monoclonal antibodies, **characterized in that** the first primary antibody is directed against the phosphorylated p24 BDV phosphoprotein and the second primary antibody is directed against p40 BDV nucleoprotein epitopes, those which do not overlap with the binding domain of the BDV phosphoprotein on the BDV nucleoprotein, for the determination of p24 phosphorylated heterodimers of p24 BDV phosphoprotein and p40 BDV nucleoprotein.

2. Method according to one of the preceding claims, **characterized in that** the immobilized primary antibody is present immobilized on a surface via a first secondary antibody or is immobilized directly on the surface.

3. Method according to one of the preceding claims, **characterized in that** the non-immobilized primary second antibody is determined via a second secondary antibody comprising a reporter molecule.

4. The method according to any one of the preceding claims, wherein the first primary antibody and the second primary antibody are from different species.

5. The method according to any one of the preceding claims, wherein the secondary antibodies are of different species or are derived from the same species.

6. Method according to one of the preceding claims, **characterized in that** circulating immune complexes (CIC) of BDV antigens, in particular of p24 protein and/or p40 protein and specific antibodies attached thereto, are additionally detected.

7. Method according to one of the preceding claims, **characterized in that** additionally circulating free antibodies against p24 protein and/or the p40 protein, in particular against phosphorylated p24 protein and against p40 protein, are detected.

8. Method according to one of the preceding claims, **characterized in that** this method is used for diagnosis and/or follow-up, in particular for differentiating acute BDV infections from chronic and dormant infections and/or their follow-up and for excluding a BDV infection, in particular in psychiatric and/or neurological diseases, as well as for determining the prevalence of infection in epidemiological studies on the mental and physical health of the population, in particular that this method, including the differentiation of acute BDV infections from chronic and dormant infections and/or their follow-up, is used for the differential diagnosis and exclusion of a BDV infection in other chronic diseases and chronic infections with other pathogens which are associated with reduced immune defense, as well as in diseases which require treatment with drugs to reduce or suppress the immune system.

9. Use of a diagnostic kit for a sandwich ELISA for the detection of acute BDV infections according to a method according to any one of claims 1 to 8, comprising a first primary antibody and a second primary antibody, wherein one of said primary antibodies is directed against the p24 BDV phosphoprotein and the other primary antibody is directed against the p40 BDV nucleoprotein; at least one secondary antibody labeled with a reporter molecule; optionally means for immobilizing a primary antibody to a surface and instructions for carrying out a method according to any one of claims 1 to 8, wherein said primary antibodies are monoclonal antibodies and wherein the first primary antibody is directed against the phosphorylated p24 BDV phosphoprotein and the second primary antibody is directed against p40 BDV nucleoprotein epitopes, those which do not overlap with the binding domain of the BDV phosphoprotein on the BDV nucleoprotein, for the determination of p24 phosphorylated heterodimers of p24 BDV phosphoprotein and p40 BDV nucleoprotein.

10. The use of a diagnostic kit for a sandwich ELISA for the detection of acute BDV infections according to claim 9, wherein the primary antibodies are as defined in claim 1 or 4 and/or wherein the secondary antibodies are as defined in any one of claims 3 and 5.

11. Use of a diagnostic kit according to any one of claims 9 or 10, further comprising means for detecting CIC from BDV antigens and specific antibodies attached thereto.

12. Use of a diagnostic kit according to any one of claims 9 to 11, further comprising means for detecting circulating free antibodies against the p24 protein and/or p40 protein, in particular the phosphorylated p24 protein and the p40 protein in plasma/serum.

13. Use of the method according to any one of claims 1 to 8 or of the diagnostic kit according to any one of claims 9 to 12 for the diagnosis and/or follow-up and/or exclusion of BDV infections, in particular in psychiatric and/or neurological diseases, for the differential diagnosis of chronic diseases and/or chronic infections of other genesis which are associated with a weakened immune defense and/or diseases in which a drug-induced weakened/suppressed immune defense is present or for determining the prevalence of infection in the healthy population.

## Revendications

1. Procédé de détection d'infections aiguës par le virus de la maladie de Borna (BDV), comprenant l'étape consistant à déterminer la présence d'hétérodimères de la phosphoprotéine p24 BDV et de la nucléoprotéine p40 BDV dans un échantillon au moyen d'un test ELISA en sandwich, dans lequel un premier anticorps primaire se trouve immobilisé et un deuxième anticorps primaire non immobilisé est ajouté après l'incubation de l'échantillon avec le premier anticorps immobilisé, et ces anticorps primaires sont des anticorps monoclonaux,
**caractérisé en ce que** le premier anticorps primaire est dirigé contre la phosphoprotéine p24 BDV phosphorylée, et le deuxième anticorps primaire est dirigé contre les épitopes de la nucléoprotéine p40 BDV qui ne chevauchent pas le domaine de liaison de la phosphoprotéine BDV sur la nucléoprotéine BDV, pour la détermination des hétérodimères phosphorylés p24 de la phosphoprotéine p24 BDV et de la nucléoprotéine p40 BDV.

2. Procédé selon la revendication précédente,
**caractérisé en ce que** l'anticorps primaire immobilisé se trouve immobilisé sur une surface par l'intermédiaire d'un premier anticorps secondaire ou est directement immobilisé sur la surface.

3. Procédé selon l'une des revendications précédentes,
**caractérisé en ce que** le deuxième anticorps primaire non immobilisé est déterminé par l'intermédiaire d'un deuxième anticorps secondaire présentant une molécule rapporteuse.

4. Procédé selon l'une des revendications précédentes,
dans lequel le premier anticorps primaire et le deuxième anticorps primaire sont issus d'espèces différentes.

5. Procédé selon l'une des revendications précédentes,
dans lequel les anticorps secondaires sont issus d'espèces différentes ou des mêmes espèces.

6. Procédé selon l'une des revendications précédentes,
**caractérisé en ce que** l'on détecte en outre des complexes immuns circulants (CIC) d'antigènes BDV, en particulier de la protéine p24 et/ou de la protéine p40 et d'anticorps spécifiques qui y sont attachés.

7. Procédé selon l'une des revendications précédentes,
**caractérisé en ce que** l'on détecte en outre des anticorps libres circulants dirigés contre la protéine p24 et/ou contre la protéine p40, en particulier contre la protéine p24 phosphorylée et contre la protéine p40.

8. Procédé selon l'une des revendications précédentes,
**caractérisé en ce que** ce procédé est utilisé pour le diagnostic et/ou le suivi, en particulier pour différencier les infections BDV aiguës des infections chroniques et dormantes, et/ou pour leur suivi et pour l'exclusion d'une infection BDV, en particulier dans le cas de maladies psychiatriques et/ou neurologiques, ainsi que pour la détermination de la prévalence de l'infection lors d'enquêtes épidémiologiques sur la santé mentale et physique de la population, en particulier, **en ce que** ce procédé, y compris la différenciation des infections BDV aiguës des infections chroniques et dormantes et/ou leur suivi, est utilisé pour le diagnostic différentiel et pour l'exclusion d'une infection BDV dans le cas d'autres maladies chroniques et d'infections chroniques par d'autres agents pathogènes qui s'accompagnent d'une diminution des défenses immunitaires, ainsi que dans le cas de maladies qui nécessitent un traitement par des médicaments destinés à diminuer ou à supprimer le système immunitaire.

9. Utilisation d'un kit de diagnostic pour un test ELISA en sandwich, destinée à détecter des infections BDV aiguës par un procédé selon l'une des revendications 1 à 8, comprenant un premier anticorps primaire et un deuxième anticorps primaire, l'un de ces anticorps primaires étant dirigé contre la phosphoprotéine p24 BDV et l'autre anticorps primaire étant dirigé contre la nucléoprotéine p40 BDV ; au moins un anticorps secondaire marqué par une molécule rapporteuse ; éventuellement des moyens pour immobiliser un anticorps primaire sur une surface, ainsi que des instructions pour la mise en œuvre d'un procédé selon l'une des revendications 1 à 8, dans laquelle lesdits anticorps primaires sont des anticorps monoclonaux, et le premier anticorps primaire est dirigé contre la phosphoprotéine p24 BDV phosphorylée et le deuxième anticorps primaire est dirigé contre les épitopes de la nucléoprotéine p40 BDV qui ne chevauchent pas le domaine de liaison de la phosphoprotéine BDV sur la nucléoprotéine BDV, pour la détermination des hétérodimères phosphorylés p24 de la phosphoprotéine p24 BDV et de la nucléoprotéine p40 BDV.

10. Utilisation d'un kit de diagnostic pour un test ELISA en sandwich, destinée à détecter des infections BDV aiguës, selon la revendication 9, dans laquelle les anticorps primaires sont tels que définis dans la revendication 1 ou 4 et/ou dans laquelle les anticorps secondaires sont tels que définis dans l'une des revendications 3 et 5.

11. Utilisation d'un kit de diagnostic selon l'une des revendications 9 ou 10, comprenant en outre des moyens de détection de CIC d'antigènes BDV et d'anticorps spécifiques qui y sont attachés.

12. Utilisation d'un kit de diagnostic selon l'une des revendications 9 à 11, comprenant en outre des moyens de détection d'anticorps libres circulants dirigés contre la protéine p24 et/ou contre la protéine p40, en particulier contre la protéine p24 phosphorylée et contre la protéine p40 dans le plasma/sérum.

13. Utilisation du procédé selon l'une des revendications 1 à 8 ou du kit de diagnostic selon l'une des revendications 9 à 12 pour le diagnostic et/ou le suivi et/ou l'exclusion d'infections BDV, en particulier dans le cas de maladies psychiatriques et/ou neurologiques, pour le diagnostic différentiel de maladies chroniques et/ou d'infections chroniques d'une autre pathogenèse qui s'accompagnent d'un affaiblissement des défenses immunitaires, et/ou de maladies dans lesquelles les défenses immunitaires sont affaiblies/supprimées par des médicaments, ou pour la détermination de la prévalence de l'infection dans la population en bonne santé.
